# EUROPÄISCHE PATENTSCHRIFT

(11) **EP 0 399 291 B1**
(45) Veröffentlichungstag und Bekanntmachung des Hinweises auf die Patenterteilung: **20.07.1994**
(21) Anmeldenummer: 90108775.9
(22) Anmeldetag: 10.05.1990
(51) Int. Cl.: C07D 215/14, A61K 31/47

(54) **Substituierte (Chinolin-2-yl-methoxy)phenyl-acyl-sulfonamide und -cyanamide, Verfahren zu ihrer Herstellung und ihre Verwendung in Arzneimitteln**
Substituted (Quinoline-2-yl-methoxy)phenyl-acyl-sulphonamides and cyanamides , process for their preparation and their use in medicines
(Quinoléine-2-yl-méthoxy)phényl-acyl-sulfonamides et cyanamides substitués, procédé pour leur préparation et leur utilisation en médicaments

(30) Priorität: 23.05.1989 DE 3916663
(43) Veröffentlichungstag der Anmeldung: 28.11.1990
(73) Patentinhaber: BAYER AG, 51368 Leverkusen (DE)
(72) Erfinder: Raddatz, Siegfried, Dr., D-5000 Köln 80 (DE); Mohrs, Klaus-Helmut, Dr., D-5600 Wuppertal 1 (DE); Fruchtmann, Romanis, Dipl.-Biol., D-5000 Köln 1 (DE); Kohlsdorfer, Christian, Dr., D-5042 Erfstadt (DE); Theisen-Popp, Pia, Dr., D-5060 Bergisch Gladbach 2 (DE); Müller-Peddinghaus,Reiner, Prof.Dr., D-5060 Bergisch Gladbach (DE)

(56) Entgegenhaltungen:
- EP-A- 0 219 308
- GB-A- 2 202 223

## Beschreibung

Die Erfindung betrifft neue substituierte (Chinolin-2-yl-methoxy)phenylacyl-sulfonamide und -cyanamide, Verfahren zu ihrer Herstellung und ihre Verwendung in Arzneimitteln.

Es ist bekannt, daß N-(Phenylmethoxy)-3-(2-chinolin-2-yl-methoxy)benzenacetamid-Derivate eine antiallergische, antiasthmatische und antiinflammatorische Wirkung besitzen [vgl. US 4 769 461]. In der EP-A2 0 219 308 werden 2-substituierte Chinolinderivate mit antiasthmatischer, antiallergischer und antiinflammatorischer Wirkung beschrieben, deren Substituentendefinition auch die Acylsulfonamidogruppe umfaßt. GB-A-2 202 223 offenbart Chinolinyl-phenoxy-sulfonylcarboxaminde, die aber in der α-Stellung nicht substituirt sind, mit lipoxygenasehemmender Wirkung

Es wurden nun substituierte (Chinolin-2-yl-methoxy)-phenylacyl-sulfonamide und -cyanamide der allgemeinen Formel (I) 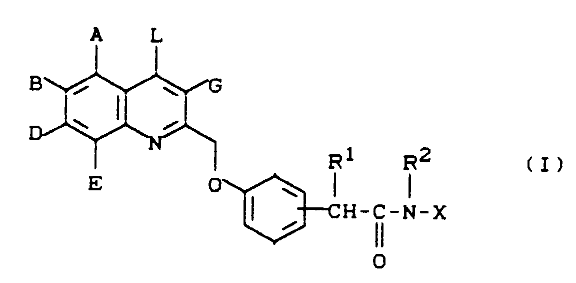
in welcher
- A, B, D, E, L und G: gleich oder verschieden sind und
- für Wasserstoff, Hydroxy, Halogen, Carboxy, Nitro, Trifluormethyl, Trifluormethoxy oder eine Gruppe der Formel -NR³R⁴ stehen,
worin
- R³ und R⁴: gleich oder verschieden sind und Wasserstoff, geradkettiges oder verzweigtes Alkyl mit bis zu 8 Kohlenstoffatomen oder Aryl mit 6 bis 10 Kohlenstoffatomen bedeuten,
- für geradkettiges oder verzweigtes Alkyl, Alkoxy oder Alkoxycarbonyl mit jeweils bis zu 12 Kohlenstoffatomen stehen, das gegebenenfalls durch Hydroxy, Halogen, Nitro, Cyano oder eine Gruppe der Formel -NR³R⁴ substituiert ist,
worin
- R³ und R⁴: die oben angegebene Bedeutung haben,
- für Aryl mit 6 bis 10 Kohlenstoffatomen stehen, das gegebenenfalls durch Halogen, Hydroxy, Nitro, Cyano, geradkettiges oder verzweigtes Alkyl, Alkoxy oder Alkoxycarbonyl mit jeweils bis zu 8 Kohlenstoffatomen oder durch eine Gruppe der Formel -NR³R⁴ substituiert ist,
worin
- R³ und R⁴: die oben angegebene Bedeutung haben,
- R¹: - für Cycloalkyl mit 3 bis 14 Kohlenstoffatomen steht das gegebenenfalls durch geradkettiges oder verzweigtes Alkyl mit bis zu 8 Kohlenstoffatomen substituiert ist,
- R²: - für Wasserstoff oder
- für geradkettiges oder verzweigtes Alkyl mit bis zu 10 Kohlenstoffatomen steht, das gegebenenfalls durch Hydroxy, Alkoxy mit bis zu 8 Kohlenstoffatomen, Halogen oder durch Cycloalkyl mit 3 bis 8 Kohlenstoffatomen oder Aryl mit 6 bis 10 Kohlenstoffatomen substituiert ist, das seinerseits durch geradkettiges oder verzweigtes Alkyl mit bis zu 8 Kohlenstoffatomen, Halogen, Nitro, Hydroxy oder Cyano substituiert sein kann, oder
- für Cycloalkyl mit 3 bis 8 Kohlenstoffatomen steht, das gegebenenfalls durch geradkettiges oder verzweigtes Alkyl mit bis zu 8 Kohlenstoffatomen substituiert ist, oder
- für ein Alkalimetall steht,
- X: - für eine Gruppe der Formel -SO₂-R⁵ steht,
worin
- R⁵: - Trifluormethyl oder geradkettiges oder verzweigtes Alkyl mit bis zu 10 Kohlenstoffatomen bedeutet, das gegebenenfalls durch Hydroxy, Halogen, Cyano, Alkoxy oder Alkoxycarbonyl mit jeweils bis zu 8 Kohlenstoffatomen oder durch Aryl mit 6 bis 10 Kohlenstoffatomen substituiert ist, welches seinerseits durch Halogen, Nitro, Cyano oder geradkettiges oder verzweigtes Alkyl oder Alkoxy mit jeweils bis zu 8 Kohlenstoffatomen substituiert sein kann, oder
- Aryl mit 6 bis 10 Kohlenstoffatomen bedeutet, das gegebenenfalls durch Halogen, Nitro, Cyano, Hydroxy, geradkettiges oder verzweigtes Alkyl, Alkoxy oder Alkoxycarbonyl mit jeweils bis zu 8 Kohlenstoffatomen, Trifluormethyl oder Trifluormethoxy substituiert ist,
oder
- X: - für Cyano steht
und deren physiologisch unbedenklichen Salze gefunden.

Im Rahmen der vorliegenden Erfindung werden physiologisch unbedenkliche Salze bevorzugt. Physiologisch unbedenkliche Salze der substituierten (Chinolin-2-yl-methoxy)phenylacyl-sulfonamide und -cyanamide können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Die erfindungsgemäßen Verbindungen können in stereoisomeren Formen vorliegen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher
- A, B, D, E, L und G: gleich oder verschieden sind und
- für Wasserstoff, Hydroxy, Fluor, Chlor, Brom, Carboxy, Nitro, Trifluormethyl, Trifluormethoxy oder eine Gruppe der Formel -NR³R⁴ stehen,
worin
- R³ und R⁴: gleich oder verschieden sind und Wasserstoff, geradkettiges oder verzweigtes Alkyl mit bis zu 6 Kohlenstoffatomen oder Phenyl bedeuten,
- für geradkettiges oder verzweigtes Alkyl, Alkoxy oder Alkoxycarbonyl mit jeweils bis zu 10 Kohlenstoffatomen stehen, das gegebenenfalls durch Hydroxy, Fluor, Chlor, Brom, Nitro, Cyano oder eine Gruppe der Formel -NR³R⁴ substituiert ist,
worin
- R³ und R⁴: die oben angegebene Bedeutung haben,
- für Phenyl stehen, das gegebenenfalls durch Fluor, Chlor, Brom, Hydroxy, Nitro, Cyano, geradkettiges oder verzweigtes Alkyl, Alkoxy oder Alkoxycarbonyl mit jeweils bis zu 6 Kohlenstoffatomen oder durch eine Gruppe der Formel -NR³R⁴ substituiert ist,
worin
- R³ und R⁴: die oben angegebene Bedeutung haben,
- R¹: - für Cyclopropyl, Cyclobutyl, Cyclopentyl, Cyclohexyl, Cycloheptyl, Cyclooctyl, Cyclodecyl oder Cyclododecyl steht, das gegebenenfalls durch geradkettiges oder verzweigtes Alkyl mit bis zu 6 Kohlenstoffatomen substituiert ist,
- R²: - für Wasserstoff oder
- für geradkettiges oder verzweigtes Alkyl mit bis zu 8 Kohlenstoffatomen steht, das gegebenenfalls durch Hydroxy, Alkoxy mit bis zu 6 Kohlenstoffatomen, Fluor, Chlor, Brom, Cyclopropyl, Cyclobutyl, Cyclopentyl, Cyclohexyl, Cycloheptyl oder Phenyl substituiert ist, das seinerseits durch geradkettiges oder verzweigtes Alkyl mit bis zu 6 Kohlenstoffatomen, Fluor, Chlor oder Brom substituiert sein kann, oder
- für Cyclopropyl, Cyclohexyl, Cyclopentyl, Cyclohexyl oder Cycloheptyl steht, das gegebenenfalls durch geradkettiges oder verzweigtes Alkyl mit bis zu 6 Kohlenstoffatomen substituiert ist, oder
- für Natrium oder Kalium steht,
- X: - für eine Gruppe der Formel -SO₂-R⁵ steht,
worin
- R⁵: - Trifluormethyl oder geradkettiges oder verzweigtes Alkyl mit bis zu 8 Kohlenstoffatomen bedeutet, das gegebenenfalls durch Fluor, Chlor, Brom, Alkoxycarbonyl mit jeweils bis zu 6 Kohlenstoffatomen oder durch Phenyl substituiert ist, welches seinerseits durch Fluor, Chlor, Brom oder durch geradkettiges oder verzweigtes Alkyl oder Alkoxy mit jeweils bis zu 6 Kohlenstoffatomen substituiert sein kann, oder
- Phenyl bedeutet, das gegebenenfalls durch Fluor, Chlor, Brom, Nitro, Cyano, geradkettiges oder verzweigtes Alkyl, Alkoxy oder Alkoxycarbonyl mit jeweils bis zu 6 Kohlenstoffatomen oder Trifluormethyl substituiert ist,
oder
- X: - für Cyano steht
und deren physiologisch unbedenklichen Salze.

Besonders bevorzugt sind Verbindungen der allgemeinen Formel (I)
in welcher
- A, B, D, E, L und G: gleich oder verschieden sind und
- für Wasserstoff, Fluor, Chlor, Brom, Nitro oder Trifluormethyl stehen,
- für Methyl, Ethyl, Propyl, Isopropyl, Butyl oder tert.Butyl stehen,
- R¹: - für Cyclopropyl, Cyclobutyl, Cyclopentyl, Cyclohexyl, Cyclooctyl, Cyclodecyl oder Cyclododecyl, Cycloheptyl steht, das gegebenenfalls durch Methyl, Ethyl, Propyl oder Isopropyl substituiert ist,
- R²: - für Wasserstoff oder
- für geradkettiges oder verzweigtes Alkyl mit bis zu 6 Kohlenstoffatomen steht, das gegebenenfalls durch Cyclopropyl, Cyclopentyl, Cyclohexyl oder Cycloheptyl substituiert ist,
- für Cyclopropyl, Cyclopentyl, Cyclohexyl oder Cycloheptyl steht, oder
- für Natrium steht,
- X: - für eine Gruppe der Formel -SO₂-R⁵ steht,
worin
- R⁵: - Trifluormethyl, geradkettiges oder verzweigtes Alkyl mit bis zu 6 Kohlenstoffatomen bedeutet, das gegebenenfalls durch Phenyl substituiert ist, welches seinerseits durch Fluor, Chlor oder durch geradkettiges oder verzweigtes Alkyl mit bis zu 4 Kohlenstoffatomen substituiert ist, oder
- Phenyl bedeutet, das gegebenenfalls durch Fluor, Chlor oder geradkettiges oder verzweigtes Alkyl mit bis zu 4 Kohlenstoffatomen substituiert sein kann,
oder
- X: - für Cyano steht
und deren physiologisch unbedenklichen Salze.

Die erfindungsgemäßen Verbindungen der allgemeinen Formel (I) 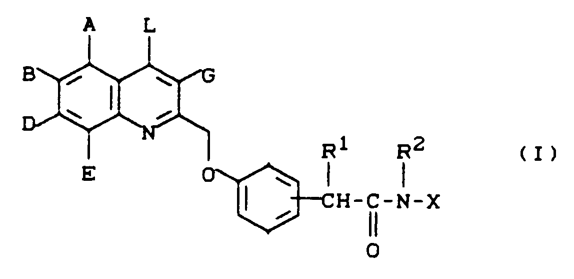
in welcher
A, B, D, E, L, G, R¹, R² und X die oben angegebene Bedeutung haben,
können hergestellt werden, indem man
Carbonsäuren der allgemeinen Formel (II) 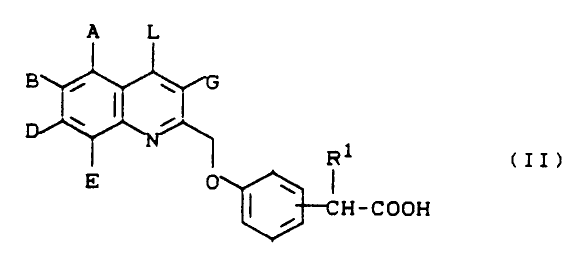
in welcher
A, B, D, E, L, G und R¹ die oben angegebene Bedeutung haben,
mit Amiden der allgemeinen Formel (III) 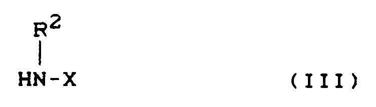
in welcher
R² und X die oben angegebene Bedeutung haben,
in inerten Lösemitteln, gegebenenfalls in Anwesenheit von Dehydratisierungsmitteln amidiert.

(Chinolin-2-yl-methoxy)phenylacyl-sulfonamide der allgemeinen Formel (Ia) 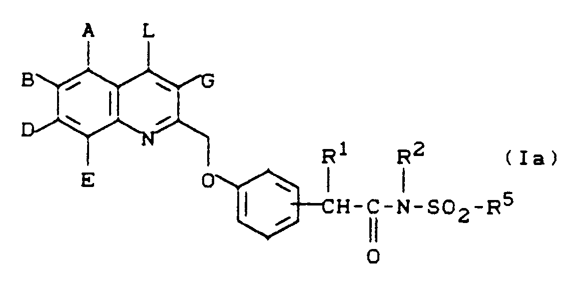
worin A, B, D, E, L, G, R¹, R² und R⁵ die oben angegebene Bedeutung haben, können auch nach einer Verfahrensvariante hergestellt werden, indem man zunächst die Carbonsäuren der allgemeinen Formel (II) über die Stufen der Säurehalogenide oder -anhydride nach üblichen Methoden in die entsprechenden Säureamide der allgemeinen Formel (IV) 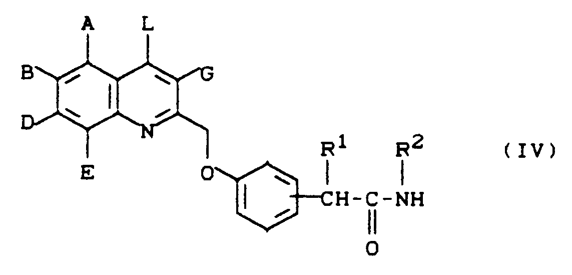
in welcher
A, B, D, E, L, G, R¹ und R² die oben angegebene Bedeutung haben,
überführt und in einem zweiten Schritt mit Sulfonsäurehalogeniden der allgemeinen Formel (V)

X-Hal (V)

in welcher
- X: - in diesem Fall für die Gruppe -SO₂-R⁵ steht,
   worin R⁵ die oben angegebene Bedeutung hat,
und
Hal für Fluor, Chlor, Brom oder Jod steht,
in inerten Lösemitteln sulfoniert.

Die erfindungsgemäßen Verfahren können durch die folgenden Formelschemata beispielhaft erläutert werden: 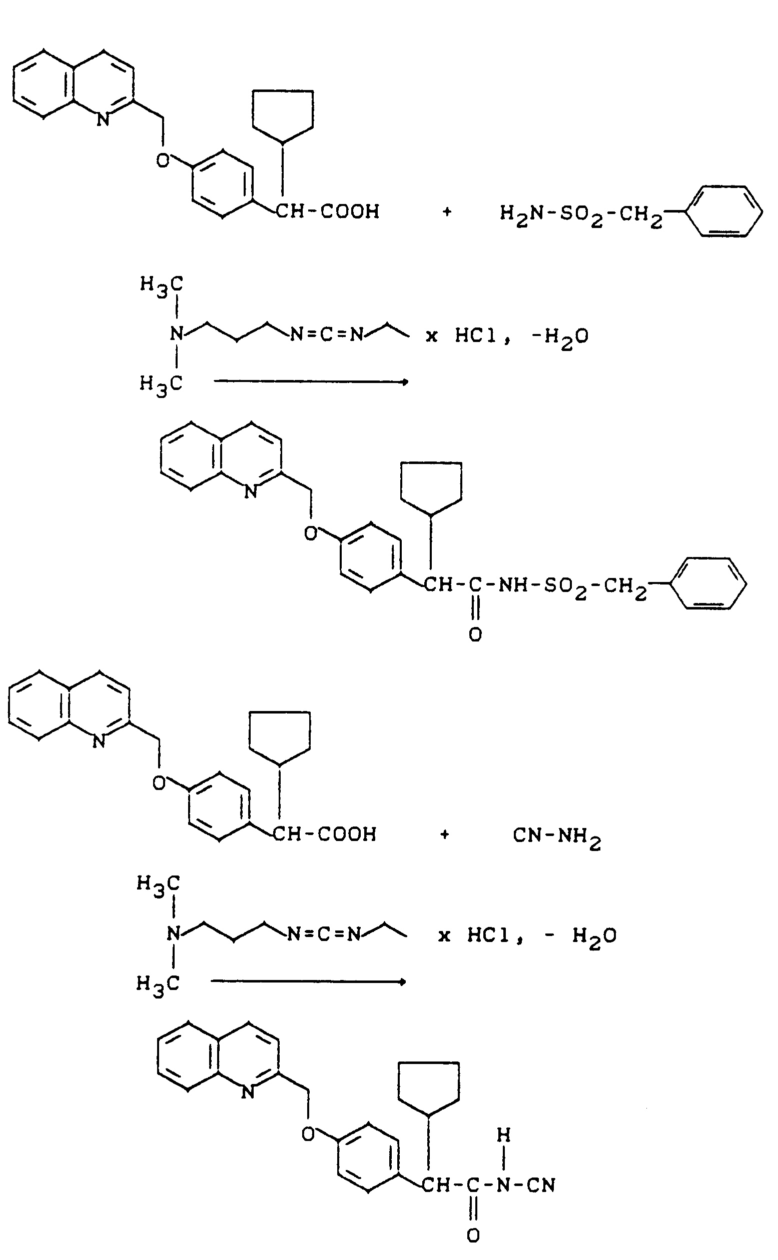 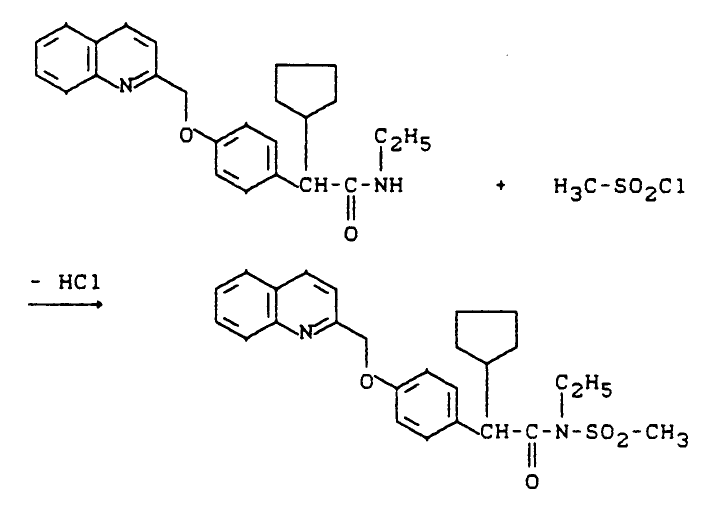
Die Amidierung der Verbindungen der allgemeinen Formel (II) erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat- oder -ethanolat oder Kalium-tert.-butylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0°C bis 150°C, bevorzugt bei 25 bis 40°C, durchgeführt.

Die Amidierung wird im allgemeinen bei Normaldruck durchgeführt. Es ist aber auch möglich, das Verfahren bei Unterdruck oder bei Überdruck durchzuführen (z. B. in einem Bereich von 0,5 bis 5 bar).

Bei der Durchführung der Amidierung wird die Base im allgemeinen in einer Menge von 1 bis 3 Mol, bevorzugt von 1 bis 1,5 Mol bezogen auf 1 Mol der Carbonsäure der allgemeinen Formel (II), eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N′-ethylcarbodiimid Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Frerman et al., J. Biol. Chem. 225, 2199, (1980) und N.B. Benoton, K. Kuroda, Int. J.Pept. Prot. Res. 13, 403 (1979), 17, 187 (1981)].

Die Sulfonierung der Verbindungen der allgemeinen Formel (IV) erfolgt in den oben erwähnten inerten Lösemitteln, gegebenenfalls unter Einsatz der ebenfalls oben aufgeführten Basen und Dehydratisierungsmittel.

Die Sulfonierung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Die Sulfonierung wird im allgemeinen in einem Temperaturbereich von 0°C bis 150°C, bevorzugt von +25°C bis +40°C, durchgeführt.

Die Verbindungen der allgemeinen Formel (III) sind bekannt oder können nach üblicher Methode hergestellt werden [vgl. Houben-Weyl, "Methoden der organischen Chemie", Band IX, S. 407 ff und J. March, Advanced Organic Chemistry, Second Edition (1977) [vgl. J. March, "Advanced Organic Chemistry", Second Edition S. 824 ff. (1977)].

Die Verbindungen der allgemeinen Formeln (II) und (IV) sind neu und können hergestellt werden, indem man beispielsweise
Verbindungen der allgemeinen Formeln (VI) oder (VII) 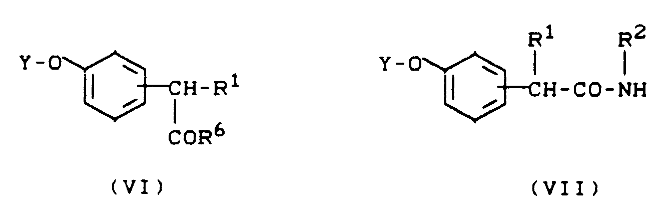
in welcher
- R¹ und R²: die oben angegebene Bedeutung haben,
- R⁶: für Hydroxy, C₁-C₆-Alkoxy oder Phenyloxy steht
und
- Y: - für eine typische Hydroxyschutzgruppe, wie beispielsweise Benzyl oder tert.Butyl steht,
nach Abspaltung der Schutzgruppe nach üblicher Methode mit Halogenmethylchinolinen der allgemeinen Formel (VIII) 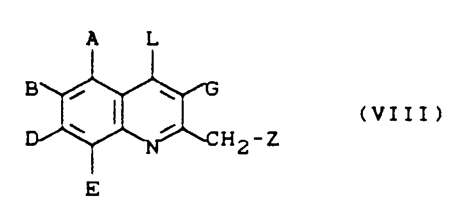
in welcher
- A, B, D, E, L und G: gleich oder verschieden sind und die oben angegebene Bedeutung haben
und
- Z: - für Halogen steht,
in inerten Lösemitteln, gegebenenfalls in Anwesenheit einer Base verethert
oder im Fall der Verbindungen der Formel (II) außerdem Verbindungen der Formel (IX) 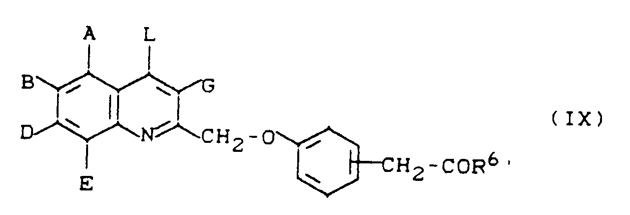
in welcher
A, B, D, E, L und G die oben angegebene Bedeutung haben
und
- R⁶: die oben angegebene Bedeutung von R⁶ hat, aber nicht für Hydroxy steht
direkt mit Verbindungen der Formel (X)

R¹-Y (X)

in welcher
- R¹: die oben angegebene Bedeutung hat und
- Y: für Halogen, vorzugsweise für Bromid steht nach üblicher Methode alkyliert,
und im Fall der Säuren die Ester verseift.

Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley & Sons, 1981, New York].

Die Veretherung kann in inerten organischen Lösemitteln gegebenenfalls in Anwesenheit einer Base durchgeführt werden.

Lösemittel für die Veretherung können inerte organische Lösemittel sein, die sich unter den Reatkionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl(C₁-C₆)amine) wie Triethylamin, oder Heterocyclen wie Pyridin, Methylpiperidin, Piperidin oder Morpholin.

Es ist auch möglich, als Basen Alkalimetalle wie Natrium, und deren Hydride, wie Natriumhdyrid, einzusetzen.

Die Veretherung erfolgt im allgemeinen in einem Temperaturbereich von 0°C bis 150°C, bevorzugt von 10°C bis 100°Cbei Normaldruck. Es ist aber auch möglich, die Veretherung bei Unterdruck oder Überdruck durchzuführen (z.B. in einem Bereich von 0,5 bis 5 bar).

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Die Alkylierung der C-H aciden Verbindungen (Formel IX) erfolgt im allgemeinen in inerten Lösemitteln in Anwesenheit einer Base mit Alkylhalogeniden.

Als Lösemittel eignen sich hierbei, je nach Art des Alkylierungsmittels alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Dimethylformamid oder Hexamethylphosphorsäuretriamid, oder Gemische der genannten Lösemittel.

Als Basen eignen sich die üblichen basischen Verbindungen. Hierzu gehören bevorzugt Alkalihydride wie Natriumhydrid, Alkaliamide wie Natriumamid oder Lithiumdiisopropylamid, Alkalialkoholate wie Natriummethanolat, Natriumethanolat, Kaliummethanolat, Kaliumethanolat oder Kalium-tert.butylat, oder organische Amine wie Trialkylamine z.B. Triethylamin, oder lithiumorganische Verbindungen wie Butyllithium oder Phenyllithium.

Die Herstellung der erfindungsgemäßen Verbindungen erfolgt im allgemeinen in einem Temperaturbereich von 0°C bis 150°C, bevorzugt von 10°C bis 100°C.

Das erfindungsgemäße Verfahren wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgemeinen in einer Menge von 0,5 bis 5 mol, bevorzugt von 1 bis 3 mol bezogen auf das Halogenid eingesetzt.

Die Verbindungen der allgemeinen Formeln (VI) und (VII) sind an sich bekannt oder können nach bekannten Methoden hergestellt werden [vgl. H. Beyer, Lehrbuch der organischen Chemie, S. Hirzel Verlag, Stuttgart; Protective Groups in Organic Synthesis, J. Wiley & Sons, 1981, New York].

Die Halogenmethylchinoline der allgemeinen Formel (VIII) sind bekannt oder können nach üblicher Methode hergestellt werden [vgl. Chem. Ber. 120, 649, 1987].

Die Verbindungen der allgemeinen Formel (X) sind bekannt. Die Verbindungen der allgemeinen Formel (IX) sind bekannt oder können nach üblichen Methoden hergestellt werden.

Zur Herstellung von isomerenreinen Verbindungen der allgemeinen Formel (I) kann selbstverständlich direkt die isomerenreine Form der Ausgangsverbindung eingesetzt werden. Sie können aber auch nach üblichen Methoden der Racematspaltung erhalten werden.

Die erfindungsgemäßen Acylsulfonamide und Acylcyanamide der allgemeinen Formel (I) können als Wirkstoffe in Arzneimitteln eingesetzt werden. Die Stoffe wirken besonders als Hemmer von enzymatischen Reaktionen im Rahmen des Arachidonsäurestoffwechsels, insbesondere der Lipoxygenase.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und/Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, Metastasen und zur Cytoprotection im Gastrointestinaltrakt geeignet.

Die pharmakologischen Wirkdaten der erfindungsgemäßen Substanzen werden durch folgende Methode bestimmt:
Als Maß für die Lipoxygenase-Hemmung wurde die Freisetzung von Leukotrien B₄ (LTB₄) an polymorphkernigen Rattenleukozyten (PMNL) nach Zugabe von Substanzen und Ca-Ionophor mittels reverse phase HPLC nach Borgeat, P. et al., Proc. Nat. Acad. Sci. 76, 2148 - 2152 (1979), bestimmt. Die in vivo-Aktivität der Verbindungen wurde mit dem Mäuseohr-Entzündungsmodell nach Young, J.M. et al., J. of Investigative Dermatology 82, 367 - 371, (1984) nachgewiesen.

In der Tabelle 1 sind beispielhaft die nach diesem Test erzielten Werte einiger erfindungsgemäßer Verbindungen aufgeführt:

**Tabelle 1**

| Beispiel | LO-Hemmung IC₅₀ (mmol/l) |
|---|---|
| 1 | 2,7 x 10⁻⁸ |
| 6 | 3,3 x 10⁻⁸ |
| 7 | 2,4 x 10⁻⁸ |

Die neuen Wirkstoffe können in an sich bekannter Weise unter Verwendung inerter nichttoxischer, pharmazeutischer geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht, zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

Die erfindungsgemäßen Arylsulfonamide und Acylcyanamide können sowohl in der Humanmedizin als auch in der Veterinärmedizin angewendet werden.

### Herstellungsbeispiele

### Beispiel 1

### N-{1-[4-(Chinolin-2-yl-methoxy)phenyl]-1-cyclopentyl}acetyl-methansulfonamid

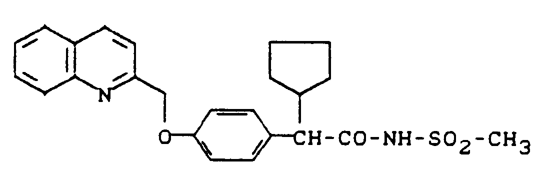

2,2 g (0,006 mol) 1-[4-(Chinolin-2-yl-methoxy)phenyl]-1-cyclopentyl-essigsäure, 0,8 g (0,006 mol) Dimethylaminopyridin, 0,6 g (0,006 mol) Methansulfonamid und 1,4 g (0,007 mol) N-(3-Dimethylaminopropyl)-N′-ethyl-carbodiimid-hydrochlorid werden in 80 ml trockenem Dichlormethan gelöst und 60 Stunden bei Raumtemperatur gerührt. Anschließend wird im Vakuum zur Trockne eingeengt, der Rückstand in 50 ml Dichlormethan suspendiert und zweimal mit 20 ml Wasser ausgeschüttelt. Die organische Phase wird getrocknet, auf ein kleines Volumen eingeengt und das Gemisch säulenchromatographisch getrennt (Kieselgel 60, Laufmittel: Dichlormethan/Essigsäureethylester/Eisessig = 100/5/1 bis 100/10/1).
Ausbeute: 1,8 g (68,4% der Theorie) eines farblosen amorphen Produktes
Fp.: ca. 75°C

### Beispiel 2

### N-{1-[4-(Chinolin-2-yl-methoxy)phenyl]-1-cylcopentyl}acetyl-benzylsulfonamid

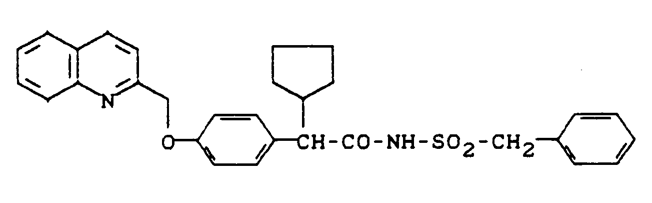

Analog der Vorschrift für Beispiel 1 wird die Titelverbindung aus 2,2 g (0,006 mol) 1-[4-(Chinolin-2-yl-methoxy)phenyl]-1-cyclopentyl-essigsäure, 0,8 g (0,006 mol) Dimethylaminopyridin, 1,1 g (0,006 mol) Benzylsulfonamid und 1,4 g (0,007 mol) N-(3-Dimethylaminopropyl)-N′-ethyl-carbodiimid-hydrochlorid hergestellt.
Ausbeute: 1,9 g (61,5% der Theorie) farblose Kristalle
Fp.: 171°C

### Beispiel 3

### N-{1-[4-(Chinolin-2-yl-methoxy)phenyl]-1-cyclopentyl}acetyl-p-tolylsulfonamid

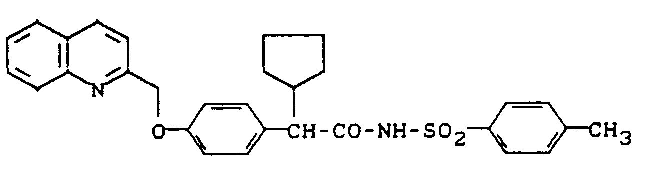

Analog der Vorschrift für Beispiel 1 wird die Titelverbindung aus 2,2 g (0,006 mol) 1-[4-(Chinolin-2-yl-methoxy)phenyl]-1-cyclopentyl-essigsäure, 0,8 g (0,006 mol) Dimethylaminopyridin, 1,1 g (0,006 mol) p-Tolylsulfonamid und 1,4 g (0,007 mol) N-(3-Dimethylaminopropyl)-N′-ethyl-carbodiimid-hydrochlorid hergestellt.
Ausbeute: 2,3 g (74,5% der Theorie) eines farblosen amorphen Produktes
Fp.: ca. 80°C

### Beispiel 4

### N-{1-[4-(Chinolin-2-yl-methoxy)phenyl]-1-cyclopentyl}acetyl-o-tolylsulfonamid

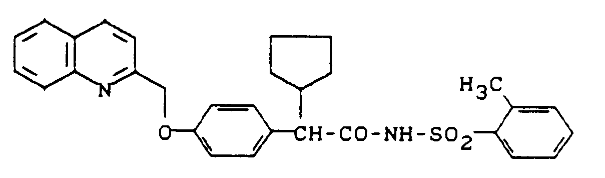

Analog der Vorschrift für Beispiel 1 wird die Titelverbindung aus 2,2 g (0,006 mol) 1-[4-(Chinolin-2-yl-methoxy)phenyl]-1-cyclopentyl-essigsäure, 0,8 g (0,006 mol) Dimethylaminopyridin, 1,1 g (0,006 mol) o-Tolylsulfonamid und 1,4 g (0,007 mol) N-(3-Dimethylaminopropyl)-N′-ethyl-carbodiimid-hydrochlorid hergestellt.
Ausbeute: 2,2 g (71,3% der Theorie) eines farblosen, amorphen Produktes
Fp.: ca. 80°C

### Beispiel 5

### N-{1-[4-(Chinolin-2-yl-methoxy)phenyl]-1-cyclopentyl}acetyl-trifluormethansulfonamid

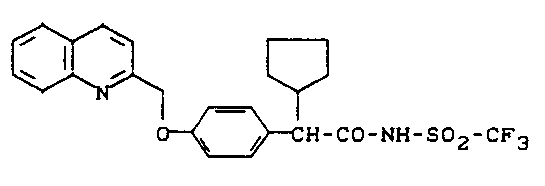

Analog der Vorschrift für Beispiel 1 wird die Titelverbindung aus 3,6 g (0,01 mol) 1-[4-(Chinolin-2-yl-methoxy)phenyl]-1-cyclopentyl-essigsäure, 1,3 g (0,01 mol) Dimethylaminopyridin, 1,9 g (0,01 mol) Trifluormethansulfonamid und 1,9 g (0,01 mol) N-(3-Dimethylaminopropyl)-N′-ethyl-carbodiimid-hydrochlorid hergestellt
Ausbeute: 3,7 g (75,1% der Theorie) farblose Kristalle
Fp.: 252°C (Zers.)

### Beispiel 6

### N-Methyl-N-1-[4-(Chinolin-2-yl-methoxy)phenyl]-1-cyclopentyl-acetyl}-trifluormethansulfonamid

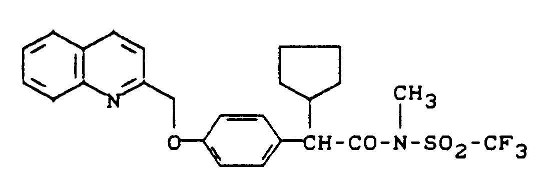

Analog der Vorschrift für Beispiel 1 wird die Titelverbindung aus 3,0 g (0,0083 mol) 1-[4-(Chinolin-2-yl-methoxy)phenyl]-1-cyclopentyl-essigsäure, 1,1 g (0,0083 mol) Dimethylaminopyridin, 1,4 g (0,0083 mol) N-Methyl-trifluormethansulfonamid und 1,6 g (0,0083 mol) N-(3-Dimethylaminopropyl)-N′-ethyl-carbodiimidhydrochlorid hergestellt.
Ausbeute: 0,6 g (14,2% der Theorie) eines farblosen Öls.

### Beispiel 7

### N-{1-[4-(Chinolin-2-yl-methoxy)phenyl]-1-cyclopentyl}acetyl-cyanamid

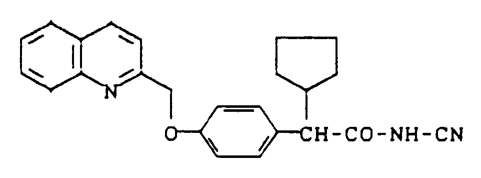

Analog der Vorschrift für Beispiel 1 wird die Titelverbindung aus 3,0 g (0,0083 mol) 1-[4-(Chinolin-2-yl-methoxy)phenyl]-1-cyclopentyl-essigsäure, 1,1 g (0,0083 mol) Dimethylaminopyridin, 0,42 g (0,0083 mol) Cyanamid und 1,6 g (0,0083 mol) N-(3-Dimethylaminopropyl)-N′-ethyl-carbodiimid-hydrochlorid hergestellt.
Ausbeute: 1,9 g (59,4% der Theorie) eines farblosen amorphen Produktes
Fp.: ca. 90°C

### Beispiel 8

### N-{1-[4-(Chinolin-2-yl-methoxy)phenyl]-1-cyclopentyl]acetyl-trifluormethansulfonamid-Natriumsalz

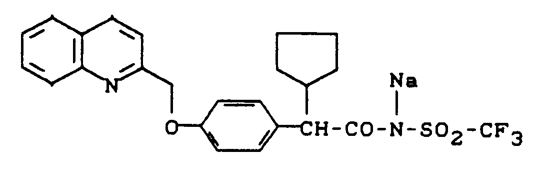

Man löste 0,374 g (0,00076 mol) der Verbindung aus Beispiel 5 in 20 ml Tetrahydrofuran/Ethanol (1:1) und tropfte 0,76 ml (0,00076 mol) 1 n Natronlauge zu der Lösung. Die Lösung ließ man 15 Minuten rühren, dampfte alles im Vakuum zur Trockne ein und trocknete im Exsikkator.
Ausbeute: quantitativ, farbloses Produkt
Fp.: 218°C (Zers.)

### Beispiel 9

### N-{1-[4-Chinolin-2-yl-methoxy)phenyl]-cyclohexyl}acetyl-methansulfonamid

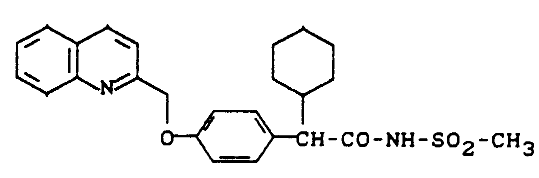

Analog zu den Angaben für Beispiel 1 wird die Titelverbindung von 7 g (0,018 mol) 1-[4-(Chinolin-2-yl-methoxy)phenyl]-1-cyclohexyl-essigsäure, 2,3 g (0,018 mol) Dimethylaminopyridin, 1,8 g (0,018 mol) Methansulfonamid und 4,8 g (0,025 mol) N-(3-Dimethylaminopropyl)-N-'ethylcarbodiimid-hydrochlorid hergestellt.
Ausbeute: 5,8 g (72 % der Theorie) eines leicht gelblichen viskosen Öls.

### Beispiel 10

### N-{1-[4-(Chinolin-2-yl-methoxy)phenyl]-cyclohexyl}acetyl-benzylsulfonamid

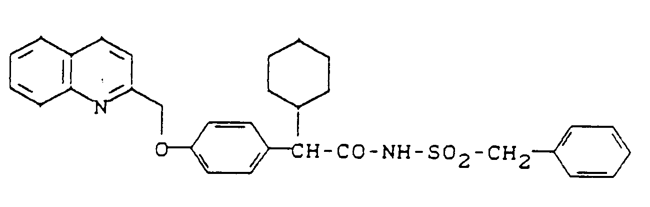

Analog zu den Angaben für Beispiel 1 wird die Titelverbindung von 2,7 g (0,0072 mol) 1-[4-(Chinolin-2-yl-methoxy)phenyl]-1-cyclohexyl-essigsäure, 1,0 g (0,0078 mol) Dimethylaminopyridin, 1,3 g (0,0072 mol) Benzylsulfonamid und 1,5 g (0,0078 mol) N-(3-Dimethylaminopropyl)-N′-ethyl-carbodiimid-hydrochlorid hergestellt.
Ausbeute: 2,8 g (74 % der Theorie) eines farblosen
Kristalls. Fp.: 123°C.

### Beispiel 11

### N-{1-[4-(Chinolin-2-yl-methoxy)phenyl]-1-cyclohexyl}acetyl-o-tolylsulfonamide

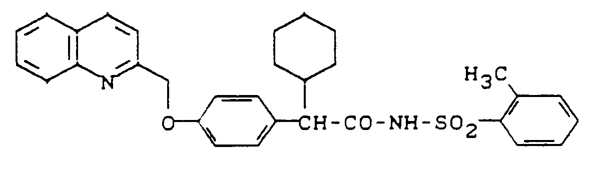

Analog zu den Angaben für Beispiel 1 wird die Titelverbindung von 2 g (0,005 mol) 1-[4-(Chinolin-2-yl-methoxy)phenyl]-1-cyclohexyl-essigsäure, 0,6 g (0,005 mol) Dimethylaminopyridin, 0,9 g (0,005 mol) o-Tolylsulfonamid und 1,15 g (0,006 mol) N-(3-Dimethylaminopropyl)-N′-ethyl-carbodiimid-hydrochloride.
Ausbeute: 2,5 g (94,6 % der Theorie) eines leicht gelblichen viskosen Öls.

### Beispiel 12

### N-{1-[4-(Chinolin-2-yl-methoxy)phenyl]-cyclohexyl}acetyl-cyanamid

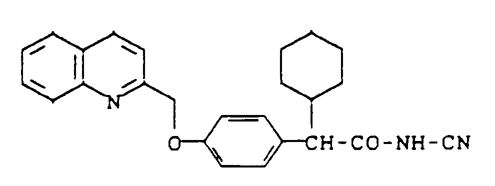

Analog zu den Angaben für Beispiel 1 wird die Titelverbindung von 3,0 g (0,008 mol) 1-[4-(Chinolin-2-yl-methoxy)phenyl]-1-cyclohexyl-essigsäure, 1,0 g Dimethylaminopyridin, 0,4 g (0,008 mol) Cyanamide und 1,6 g (0,008 mol) N-(3-Dimethylaminopropyl)-N′-ethyl-carbodiimid-hydrochlorid.
Ausbeute: 2,0 g (62 % der Theorie) eines farblosen
amorphen Produkts: Fp.: ca. 90°C.

### Beispiel 13

### N-{1-[4-(Chinolin-2-yl-methoxy)phenyl]-1-cycloheptyl}acetyl-methansulfonamid

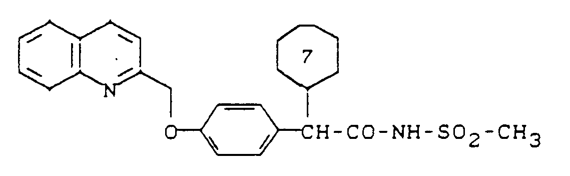

Analog zu den Angaben für Beispiel 1 wird die Titelverbindung von 8 g (0,02 mol) 1-[4-(Chinolin-2-yl-methoxy)phenyl]-1-cycloheptyl-essigsäure, 2,4 g (0,02 mol) Dimethylaminopyridin, 1,9 g (0,02 mol) Methansulfonamid und 5,7 g (0,03 mol) N-(3-Dimethylaminopropyl)-N′-ethyl-carbodiimid-hydrochlorid.
Ausbeute: 6,3 g (73 % der Theorie) farbloses Kristall.
Fp.: 174-6°C.

### Beispiel 14

### N-{1-[4-(Chinolin-2-yl-methoxy)phenyl]-1-cycloheptyl}acetyl-trifluormethansulfonamid

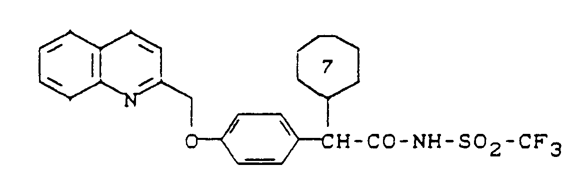

Analog zu den Angaben für Beispiel 1 wird die Titelverbindung von 5,0 g (0,013 mol) 1-[4-(Chinolin-2-yl-methoxy)phenyl]-1-cycloheptyl-essigsäure, 2,5 g (0,02 mol) Dimethylaminopyridin, 2,0 g (0,013 mol) Trifluormethansulfonamid und 3,8 g (0,02 mol) N-(3-Dimethylaminopropyl)-N′-ethyl-carbodiimid-hydrochlorid.
Ausbeute: 5,0 g (75 % der Theorie) farbloses Kristall.
Fp.: 210°C (Zers.).

### Beispiel 15

### N-{1-[4-(Chinolin-2-yl-methoxy)phenyl]-1-cycloheptyl}acetyl-o-tolylsulfonamid

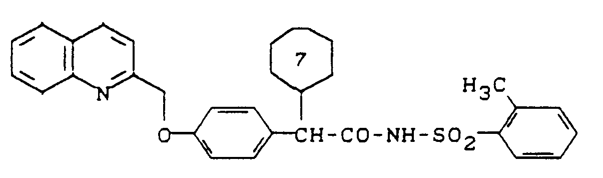

Analog zu den Angaben für Beispiel 1 wird die Titelverbindung von 4 g (0,0074 mol) 1-[4-(Chinolin-2-yl-methoxy)phenyl]-1-cycloheptyl-essigsäure, 0,9 g (0,0074 mol) Dimethylaminopyridin, 1,25 g (0,0074 mol) o-Tolylsulfonamid und 1,5 g (0,008 mol) N-(3-Dimethylaminopropyl)-N′-ethyl-carbodiimid-hyrochlorid.
Ausbeute: 2,4 g (60 % der Theorie) farbloses Kristall.
Fp.: 83-5°C.

### Beispiel 16

### N-{1-[4-(Chinolin-2-yl-methoxy)phenyl]-cyclooctyl}acetyl-methansulfonamide

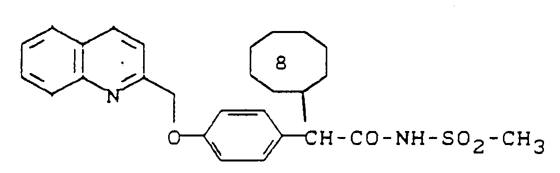

Analog zu den Angaben für Beispiel 1 wird die Titelverbindung von 1,9 g (0,0047 mol) 1-[4-(Chinolin-2-yl-methoxy)phenyl]-1-cyclooctyl-essigsäure, 0,7 g (0,0047 mol) Dimethylaminopyridin, 0,5 g (0,0047 mol) Methansulfonamid und 1,1 g (0,0057 mol) N-(3-Dimethylaminopropyl)-N′-ethyl-carbodiimid-hydrochlorid.
Ausbeute: 1,0 g (44 % der Theorie) farbloses amorphes Produkt, kein Fp.

### Beispiel 17

### N-{1-[4-(Chinolin-2-yl-methoxy)phenyl]-cyclodecyl}acetyl-methansulfonamid

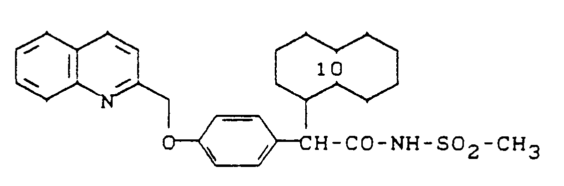

Analog zu den Angaben für Beispiel 1 wird die Titelverbindung von 0,8 g (0,00185 mol) 1-[4-(Chinolin-2-yl-methoxy)phenyl]-1-cyclodecyl-essigsäure, 0,35 g (0,0026 mol) Dimethylaminopyridin, 0,2 g (0,0021 mol) Methansulfonamid und 0,5 g (0,0026 mol) N-(3-Dimethylaminopropyl)-N′-ethyl-carbodiimid-hydrochlorid.
Ausbeute: 0,4 g (42,5 % der Theorie) farbloses amorphes Produkt, kein Fp.

### Beispiel 18

### N-{1-[4-(Chinolin-2-yl-methoxy)phenyl]-1-cyclododecyl}acetyl-methansulfonamid

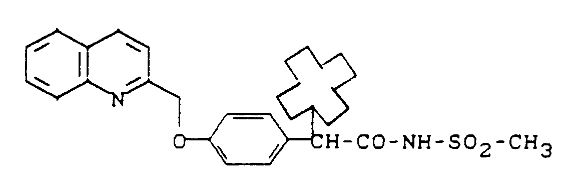

Analog zu den Angaben für Beispiel 1 wird die Titelverbindung von 1,4 g (0,003 mol) 1-[4-(Chinolin-2-yl-methoxy)phenyl]-1-cyclododecyl-essigsäure, 0,5 g (0,0036 mol) Dimethylaminopyridin, 0,3 g (0,003 mol) Methansulfonamid und 0,7 g (0,0036 mol) N-(3-Dimethylaminopropyl)-N′-ethyl-carbodiimid-hydrochlorid.
Ausbeute: 0,9 g (56 % der Theorie) farbloses amorphes Produkt, kein Fp.

### Ausgangsverbindungen (Formel II)

### Beispiel I

### N-{1-[4-(Chinolin-2-yl-methoxy)phenyl]-1-cyclooctylessigsäure

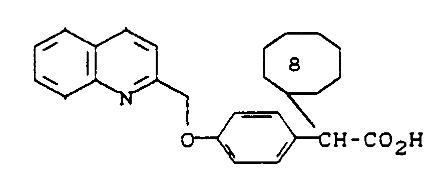

2,9 g (0,0069 mol) 1-[4-Chinolin-2-yl-methoxy)-phenyl]-1-cyclooctyl-essigsäuremethylester wurden in einem Gemisch (50:50) von Isopropanol und Dioxan und 15 ml 1n NaCO₃ über Nacht zum Sieden erhitzt. Nach dem Abkühlen versetzte man mit 15 ml 1n Salzsäure und filtrierte den angefallenen Niederschlag ab.
Ausbeute: 2,8 g (quantitativ) farblose Kristalle
m.p.: 157°C.

### Beispiel II

### 1-[4-(Chinolin-2-yl-methoxy)phenyl]-1-cyclooctylessigsäure-methylester

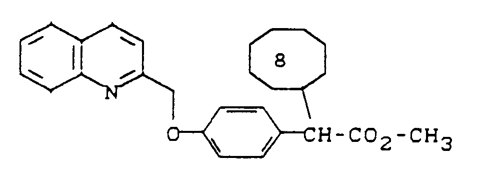

Man suspendierte 0,6 g (80 %-ig =̂ 0,02 mol) Natriumhydrid in 20 ml getrocknetem DMF unter Argon, tropfte 6,1 g (0,02 mol) 4-(Chinolin-2-yl methoxy)phenylessigsäureester in 50 ml getrocknetem DMF hinzu und erwärmte unter Rühren langsam 1 Stunde auf 25°C, wobei Gasentwicklung auftritt. Dann tropfte man 5,8 g (0,03 mol) Cyclooctylbromid zu, wobei die Temperatur auf 35°C anstieg. Anschließend ließ man noch über Nacht nachreagieren, wobei die Temperatur auf Raumtemperatur abfiel. Man versetzte mit 20 ml 1n Salzsäure, engte zur Trockne ein und rührte den Rückstand mit 50 ml Dichlormethan aus (gelindes Erwärmen). Die Dichlormethanphase schüttelte man mit 1n NaHCO₃-Lösung aus, trocknete sie mit Natriumsulfat, engte auf ein übliches Volumen ein und trennte den Rest säulenchromatographisch.
Toluol/Essigsäureethylester = 9:1, Kieselgel 60.
Ausbeute: 2,8 g (25 % der Theorie) farblose Kristalle
Fp.: 90°C.

### Beispiel III

### 1-[4-(Chinolin-2-yl-methoxy)phenyl]-1-cyclodecylessigsäure

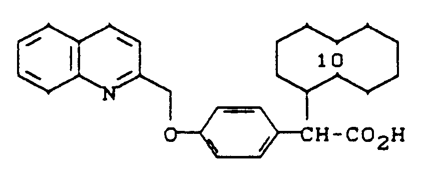

Die Titelverbindung wird in Analogie zur Vorschrift des Beispiels I aus 1,9 g (0,0043 mol) 1-[4-(Chinolin-2-yl-methoxy)phenyl]-1-cyclodecyl-essigsäuremethylester und 10 ml 1n Natronlauge in 30 ml Isopropanol hergestellt.
Ausbeute: 1,6 g (86 % der Theorie) farblose Kristalle.
Fp.: 158°C.

### Beispiel IV

### 1-[4-(Chinolin-2-yl-methoxy)phenyl]-1-cyclodecylessigsäure-methylester

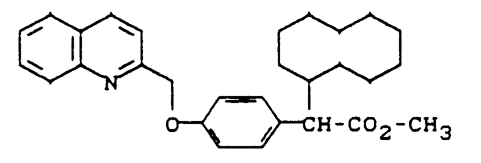

Die Titelverbindung wurde in Analogie zur Vorschrift des Beispiel II aus 6,1 g (0,02 mol) 4-(Chinolin-2-yl-methoxy)-phenyl-essigsäureethylester und 6,6 g (0.03 mol) Cyclodecylbromid hergestellt.
Ausbeute: 1,9 g (21 % der Theorie) eines leicht gelblichen Öls.

### Beispiel V

### 1-[4-(Chinolin-2-yl-methoxy)phenyl]-1-cyclododecylessigsäure

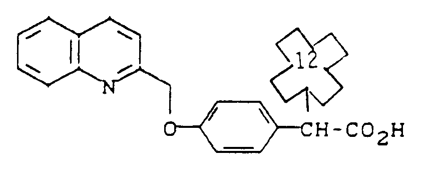

Die Titelverbindung wird in Analogie zur Vorschrift des Beispiels I aus 2,4 g (0,00507 mol) 1-[4-(chinolin-2-yl-methoxy)phenyl]-1-cyclododecyl-essigsäuremethylester und 15 ml 1n Natronlauge in 30 ml Isopropanol hergestellt.
Ausbeute: 2,3 g farblose Kristalle, Fp.: 201°C.

### Beispiel VI

### 1-[4-(Chinolin-2-yl-methoxy)phenyl]-1-cyclododecylessigsäuremethylester

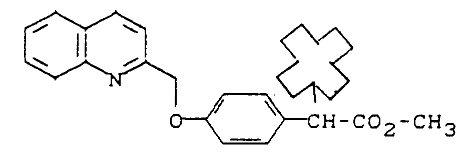

Die Titelverbindung wurde in Analogie zur Vorschrift des Beispiels II aus 6,1 g (0,002 mol) 4-(Chinolin-2-yl-methoxy)phenylessigsäuremethylester und 7,5 g (0,003 mol) Cyclododecylbromid hergestellt.

### Beispiel VII und VIII

### (+)-N-[1-[4-Chinolin-2-yl-methoxy)-phenyl]-1-cycloheptyl]acetyl-methan-sulfonamid und

### (-)-N-[1-[4-chinolin-2-yl-methoxy)-phenyl]-1-cycloheptyl]-acetyl-methansulfonamid

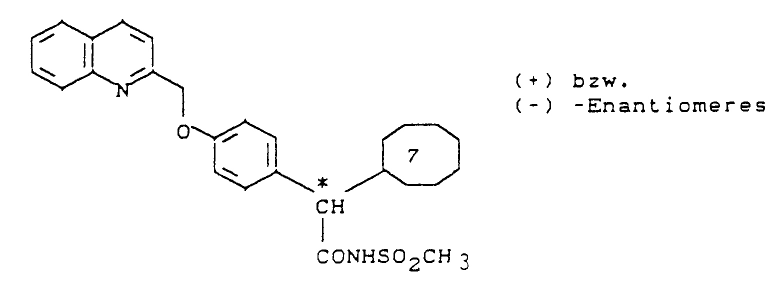

Analog der Vorschrift für Beispiel 1 werden die Titelverbindungen aus den entsprechenden enantiomeren Essigsäuren hergestellt.
(+)-Enantiomer:
Fp: 172°C
α_{D} = + 14,24°C
(-)-Enantiomer:
Fp: 172°C
α_{D} = -13,64°C
Ausgangsverbindungen:
(+)-[1-[4-(Chinolin-2-yl-methoxy)-phenyl]-1-cycloheptyl]-essigsäure
α_{D} = + 33,2°C, C = 0,8 (Aceton)
(-)-[1-[4-Chinolin-2-yl-methoxy)phenyl]-1-cylcoheptyl]-essigsäure
α_{D} = -32,2°C, C = 0,7 (Aceton)

## Patentansprüche (Patentansprüche für folgende(n) Vertragsstaat(en): AT, BE, CH, DE, DK, FR, GB, GR, IT, LI, NL, LU, SE)

1. (Chinolin-2-yl-methoxy)phenylacyl-sulfonamide und -cyanamide der allgemeinen Formel 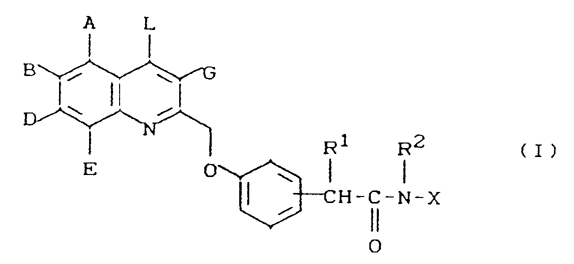 in welcher
A, B, D, E, L und G gleich oder verschieden sind und - für Wasserstoff, Hydroxy, Halogen, Carboxy, Nitro, Trifluormethyl, Trifluormethoxy oder eine Gruppe der Formel -NR³R⁴ stehen,
worin
R³ und R⁴ gleich oder verschieden sind und Wasserstoff, geradkettiges oder verzweigtes Alkyl mit bis zu 8 Kohlenstoffatomen oder Aryl mit 6 bis 10 Kohlenstoffatomen bedeuten,
- für geradkettiges oder verzweigtes Alkyl, Alkoxy oder Alkoxycarbonyl mit jeweils bis zu 12 Kohlenstoffatomen stehen, das gegebenenfalls durch Hydroxy, Halogen, Nitro, Cyano oder eine Gruppe der Formel -NR³R⁴ substituiert ist,
worin
R³ und R⁴ die oben angegebene Bedeutung haben,
- für Aryl mit 6 bis 10 Kohlenstoffatomen stehen, das gegebenenfalls durch Halogen, Hydroxy, Nitro, Cyano geradkettiges oder verzweigtes Alkyl, Alkoxy oder Alkoxycarbonyl mit jeweils bis zu 8 Kohlenstoffatomen oder durch eine Gruppe der Formel -NR³R⁴ substituiert ist,
worin
R³ und R⁴ die oben angegebene Bedeutung haben,
R¹
- für Cycloalkyl mit 3 bis 14 Kohlenstoffatomen steht, das gegebenenfalls durch geradkettiges oder verzweigtes Alkyl mit bis zu 8 Kohlenstoffatomen substituiert ist,
R²
- für Wasserstoff oder
- für geradkettiges oder verzweigtes Alkyl mit bis zu 10 Kohlenstoffatomen steht, das gegebenenfalls durch Hydroxy, Alkoxy mit bis zu 8 Kohlenstoffatomen, Halogen oder durch Cycloalkyl mit 3 bis 8 Kohlenstoffatomen oder Aryl mit 6 bis 10 Kohlenstoffatomen substituiert ist, das seinerseits durch geradkettiges oder verzweigtes Alkyl mit bis zu 8 Kohlenstoffatomen, Halogen, Nitro, Hydroxy oder Cyano substituiert sein kann, oder
- für Cycloalkyl mit 3 bis 8 Kohlenstoffatomen steht, das gegebenenfalls durch geradkettiges oder verzweigtes Alkyl mit bis zu 8 Kohlenstoffatomen substituiert ist, oder
- für ein Alkalimetall steht,
X
- für eine Gruppe der Formel -SO₂-R⁵ steht,
worin
R⁵
- Trifluormethyl oder geradkettiges oder verzweigtes Alkyl mit bis zu 10 Kohlenstoffatomen bedeutet, das gegebenenfalls durch Hydroxy, Halogen, Cyano, Alkoxy oder Alkoxycarbonyl mit jeweils bis zu 8 Kohlenstoffatomen oder durch Aryl mit 6 bis 10 Kohlenstoffatomen substituiert ist, welches seinerseits durch Halogen, Nitro, Cyano oder geradkettiges oder verzweigtes Alkyl oder Alkoxy mit jeweils bis zu 8 Kohlenstoffatomen substituiert sein kann, oder
- Aryl mit 6 bis 10 Kohlenstoffatomen bedeutet, das gegebenenfalls durch Halogen, Nitro, Cyano, Hydroxy, geradkettiges oder verzweigtes Alkyl, Alkoxy oder Alkoxycarbonyl mit jeweils bis zu 8 Kohlenstoffatomen, Trifluormethyl oder Trifluormethoxy substituiert ist,
oder
X
- für Cyano steht
und deren physiologisch unbedenklichen Salze.

2. (Chinolin-2-yl-methoxy)phenylacyl-sulfonamide und -cyanamide der Formel (I) nach Anspruch 1
worin
A, B, D, E, L und G gleich oder verschieden sind und
- für Waserstoff, Hydroxy, Fluor, Chlor, Brom, Carboxy, Nitro, Trifluormethyl, Trifluormethoxy oder eine Gruppe der Formel -NR³R⁴ stehen,
worin
R³ und R⁴ gleich oder verschieden sind und Wasserstoff, geradkettiges oder verzweigtes Alkyl mit bis zu 6 Kohlenstoffatomen oder Phenyl bedeuten,
- für geradkettiges oder verzweigtes Alkyl, Alkoxy oder Alkoxycarbonyl mit jeweils bis zu 10 Kohlenstoffatomen stehen, das gegebenenfalls durch Hydroxy, Fluor, Chlor, Brom, Nitro, Cyano oder eine Gruppe der Formel -NR³R⁴ substituiert ist,
worin
R³ und R⁴ die oben angegebene Bedeutung haben,
- für Phenyl stehen, das gegebenenfalls durch Fluor, Chlor, Brom, Hydroxy, Nitro, Cyano, geradkettiges oder verzweigtes Alkyl, Alkoxy oder Alkoxycarbonyl mit jeweils bis zu 6 Kohlenstoffatomen oder durch eine Gruppe der Formel -NR³R⁴ substituiert ist,
worin
R³ und R⁴ die oben angegebene Bedeutung haben,
R¹
- für Cyclopropyl, Cyclobutyl, Cyclopentyl, Cyclohexyl, Cycloheptyl, Cyclooctyl, Cyclodecyl oder Cyclododecyl steht, das gegebenenfalls durch geradkettiges oder verzweigtes Alkyl mit bis zu 6 Kohlenstoffatomen substituiert ist,
R²
- für Wasserstoff oder
- für geradkettiges oder verzweigtes Alkyl mit bis zu 8 Kohlenstoffatomen steht, das gegebenenfalls durch Hydroxy, Alkoxy mit bis zu 6 Kohlenstoffatomen, Fluor, Chlor, Brom, Cyclopropyl, Cyclobutyl, Cyclopentyl, Cyclohexyl, Cycloheptyl oder Phenyl substituiert ist, das seinerseits durch geradkettiges oder verzweigtes Alkyl mit bis zu 6 Kohlenstoffatomen, Fluor, Chlor oder Brom substituiert sein kann, oder
- für Cyclopropyl, Cyclohexyl, Cyclopentyl, Cyclohexyl oder Cycloheptyl steht, das gegebenenfalls durch geradkettiges oder verzweigtes Alkyl mit bis zu 6 Kohlenstoffatomen substituiert ist, oder
- für Natrium oder Kalium steht,
X
- für eine Gruppe der Formel -SO₂-R⁵ steht,
worin
R⁵
- Trifluormethyl oder geradkettiges oder verzweigtes Alkly mit bis zu 8 Kohlenstoffatomen bedeutet, das gegebenenfalls durch Fluor, Chlor, Brom, Alkoxycarbonyl mit jeweils bis zu 6 Kohlenstoffatomen oder durch Phenyl substituiert ist, welches seinerseits durch Fluor, Chlor, Brom oder durch geradkettiges oder verzweigtes Alkyl oder Alkoxy mit jeweils bis zu 6 Kohlenstoffatomen substituiert sein kann, oder
- Phenyl bedeutet, das gegebenenfalls durch Fluor, Chlor, Brom, Nitro, Cyano, geradkettiges oder verzweigtes Alkyl, Alkoxy oder Alkoxycarbonyl mit jeweils bis zu 6 Kohlenstoffatomen oder Trifluormethyl substituiert ist,
oder
X
- für Cyano steht
und deren physiologisch unbedenkliche Salze.

3. (Chinolin-2-yl-methoxy)phenylacyl-sulfonamide und -cyanamide der Formel (I) nach Anspruch 1
worin
A, B, D, E, L und G gleich oder verschieden sind und
- für Wasserstoff, Fluor, Chlor, Brom, Nitro oder Trifluormethyl stehen,
- für Methyl, Ethyl, Propyl, Isopropyl, Butyl oder tert.Butyl stehen,
R¹
- für Cyclopropyl, Cyclobutyl, Cyclopentyl, Cyclohexyl, Cycloheptyl, Cyclooctyl, Cyclodecyl oder Cyclododecyl steht, das gegebenenfalls durch Methyl, Ethyl, Propyl oder Isopropyl substituiert ist,
R²
- für Wasserstoff oder
- für geradkettiges oder verzweigtes Alkyl mit bis zu 6 Kohlenstoffatomen steht, das gegebenenfalls durch Cyclopropyl, Cyclopentyl, Cyclohexyl oder Cycloheptyl substituiert ist,
- für Cyclopropyl, Cyclopentyl, Cyclohexyl oder Cycloheptyl steht, oder
- für Natrium steht,
X
- für eine Gruppe der Formel -SO₂-R⁵ steht,
worin
R⁵
- Trifluormethyl, geradkettiges oder verzweigtes Alkyl mit bis zu 6 Kohlenstoffatomen bedeutet, das gegebenenfalls durch Phenyl substituiert ist, welches seinerseits durch Fluor, Chlor oder durch geradkettiges oder verzweigtes Alkyl mit bis zu 4 Kohlenstoffatomen substituiert ist, oder
- Phenyl bedeutet, das gegebenenfalls durch Fluor, Chlor oder geradkettiges oder verzweigtes Alkyl mit bis zu 4 Kohlenstoffatomen substituiert sein kann,
oder
X
- für Cyano steht
und deren physiologisch unbedenkliche Salze.

4. (Chinolin-2-yl-methoxy)phenylacyl-sulfonamide und -cyanamide nach Anspruch 1-3 zur Bekämpfung von Krankheiten.

5. Verfahren zur Herstellung von (Chinolin-2-yl-methoxy)phenylacyl-sulfonamiden und -cyanamiden der allgemeinen Formel 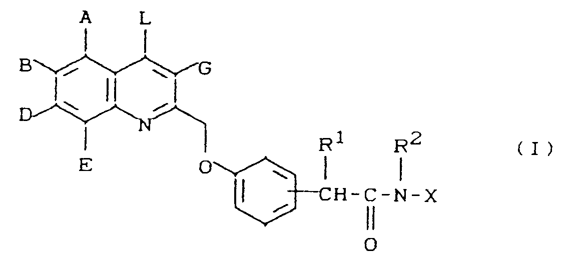 in welcher
A, B, D, E, L, G, R¹, R² und X die in Anspruch 1 bis 3 angegebene Bedeutung haben,
und deren physiologisch unbedenkliche Salze.
dadurch gekennzeichnet, daß man
Carbonsäuren der allgemeinen Formel (II) 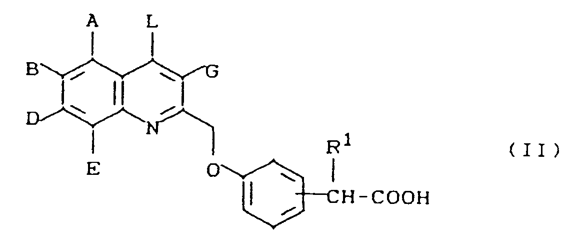 mit Amiden der allgemeinen Formel (III) 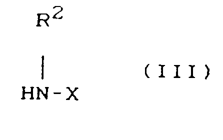 in inerten Lösemitteln, gegebenenfalls in Anwesenheit von Dehydratisierungsmitteln, amidiert.

6. Verfahren zur Herstellung von (Chinolin-2-yl-methoxy)phenylacyl-sulfonamiden der allgemeinen Formel (Ia) 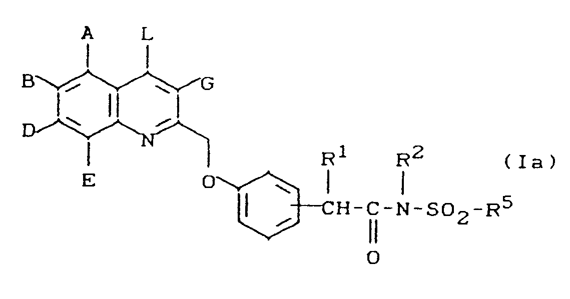 in welcher
A, B, D, E, L, G, R¹, R² und X die in Anspruch 1-3 angegebene Bedeutung haben,
X jedoch nicht für Cyano steht
und deren physiologisch unbedenkliche Salze, dadurch gekennzeichnet, daß man
zunächst die Carbonsäuren der allgemeinen Formel (II) über die Stufen der Säurehalogenide oder -anhydride nach üblichen Methoden in die entsprechenden Säureamide der allgemeinen Formel (IV) 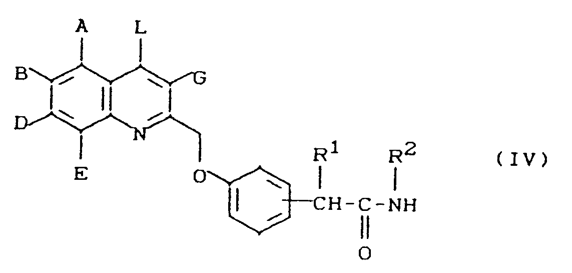 überführt und in einem zweiten Schritt mit Sulfonsäurehalogeniden der allgemeinen Formel (V)
X-Hal (V)
in welcher
X
- in diesem Fall für die Gruppe -SO₂R⁵ steht, worin R⁵ die oben angegebene Bedeutung hat,
und Hal für Fluor, Chlor, Brom oder Jod steht,
in inerten Lösemitteln sulfoniert.

7. Arzneimittel enthaltend mindestens ein (Chinolin-2-yl-methoxy)phenylacyl-sulfonamid und -cyanamid nach Anspruch 1-3.

8. Verfahren zur Herstellung eines Arzneimittels nach Anspruch 6, dadurch gekennzeichnet, daß man Verbindungen nach Anspruch 1, gegebenenfalls mit Hilfe von üblichen Hilfs- und Trägerstoffen in eine geeignete Applikationsform bringt.

9. Verwendung der (Chinolin-2-yl-methoxy)-phenylacyl-sulfonamide und -cyanamide nach Anspruch 1 zur Herstellung von Arzneimitteln.

10. (+)-Enantiomere und (-)-Enantiomere der substituierten (Chinolin-2-yl-methoxy)phenylarylsulfonamide und-cyanamide nach Anspruch 1-3.

11. (+)-N-[1-[4-Chinolin-2-yl-methoxy)-phenyl]-1-cycloheptyl]acetyl-methansulfonamid.

12. (-)-N-[1-[4-(Chinolin-2-yl-methoxy)-phenyl]-1-cycloheptyl]acetyl-methan-sulfonamid.

## Patentansprüche (Patentansprüche für folgende(n) Vertragsstaat(en): ES)

1. Verfahren zur Herstellung von (Chinolin-2-yl-methoxy)phenylacyl-sulfonamiden und -cyanamiden der allgemeinen Formel 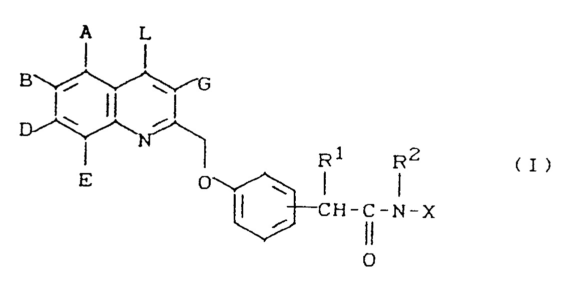 in welcher
A, B, D, E, L und G gleich oder verschieden sind und
- für Wasserstoff, Hydroxy, Halogen, Carboxy, Nitro, Trifluormethyl, Trifluormethoxy oder eine Gruppe der Formel -NR³R⁴ stehen,
worin
R³ und R⁴ gleich oder verschieden sind und Wasserstoff, geradkettiges oder verzweigtes Alkyl mit bis zu 8 Kohlenstoffatomen oder Aryl mit 6 bis 10 Kohlenstoffatomen bedeuten,
- für geradkettiges oder verzweigtes Alkyl, Alkoxy oder Alkoxycarbonyl mit jeweils bis zu 12 Kohlenstoffatomen stehen, das gegebenenfalls durch Hydroxy, Halogen, Nitro, Cyano oder eine Gruppe der Formel -NR³R⁴ substituiert ist,
worin
R³ und R⁴ die oben angegebene Bedeutung haben,
- für Aryl mit 6 bis 10 Kohlenstoffatomen stehen, das gegebenenfalls durch Halogen, Hydroxy, Nitro, Cyano geradkettiges oder verzweigtes Alkyl, Alkoxy oder Alkoxycarbonyl mit jeweils bis zu 8 Kohlenstoffatomen oder durch eine Gruppe der Formel -NR³R⁴ substituiert ist,
worin
R³ und R⁴ die oben angegebene Bedeutung haben,
R¹
- für Cycloalkyl mit 3 bis 14 Kohlenstoffatomen steht, das gegebenenfalls durch geradkettiges oder verzweigtes Alkyl mit bis zu 8 Kohlenstoffatomen substituiert ist,
R²
- für Wasserstoff oder
- für geradkettiges oder verzweigtes Alkyl mit bis zu 10 Kohlenstoffatomen steht, das gegebenenfalls durch Hydroxy, Alkoxy mit bis zu 8 Kohlenstoffatomen, Halogen oder durch Cycloalkyl mit 3 bis 8 Kohlenstoffatomen oder Aryl mit 6 bis 10 Kohlenstoffatomen substituiert ist, das seinerseits durch geradkettiges oder verzweigtes Alkyl mit bis zu 8 Kohlenstoffatomen, Halogen, Nitro, Hydroxy oder Cyano substituiert sein kann, oder
- für Cycloalkyl mit 3 bis 8 Kohlenstoffatomen steht, das gegebenenfalls durch geradkettiges oder verzweigtes Alkyl mit bis zu 8 Kohlenstoffatomen substituiert ist, oder
- für ein Alkalimetall steht,
X
- für eine Gruppe der Formel -SO₂-R⁵ steht,
worin
R⁵
- Trifluormethyl oder geradkettiges oder verzweigtes Alkyl mit bis zu 10 Kohlenstoffatome bedeutet, das gegebenenfalls durch Hydroxy, Halogen, Cyano, Alkoxy oder Alkoxycarbonyl mit jeweils bis zu 8 Kohlenstoffatomen oder durch Aryl mit 6 bis 10 Kohlenstoffatomen substituiert ist, welches seinerseits durch Halogen, Nitro, Cyano oder geradkettiges oder verzweigtes Alkyl oder Alkoxy mit jeweils bis zu 8 Kohlenstoffatomen substituiert sein kann, oder
- Aryl mit 6 bis 10 Kohlenstoffatomen bedeutet, das gegebenenfalls durch Halogen, Nitro, Cyano, Hydroxy, geradkettiges oder verzweigtes Alkyl, Alkoxy oder Alkoxycarbonyl mit jeweils bis zu 8 Kohlenstoffatomen, Trifluormethyl oder Trifluormethoxy substituiert ist,
oder
X
- für Cyano steht
und deren physiologisch unbedenkliche Salze.
dadurch gekennzeichnet, daß man
Carbonsäuren der allgemeinen Formel (II) 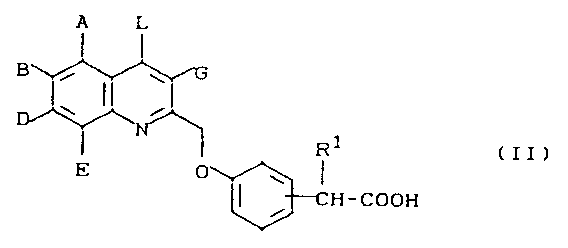 in welcher
A, B, D, E, L, G und R¹ die oben angegebene Bedeutung haben,
mit Amiden der allgemeinen Formel (III) 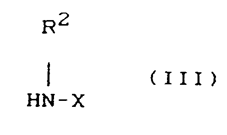 in welcher
R² und X die oben angegebene Bedeutung haben,
in inerten Lösemitteln, gegebenenfalls in Anwesenheit von Dehydratisierungsmitteln, amidiert.

2. Verfahren zur Herstellung von (Chinolin-2-yl-methoxy)phenylacyl-sulfonamide der allgemeinen Formel (Ia) 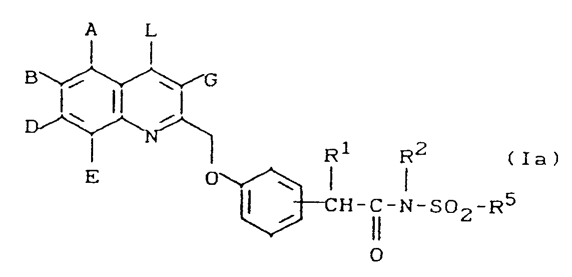 in welcher
A, B, D, E, L und G gleich oder verschieden sind und - für Wasserstoff, Hydroxy, Halogen, Carboxy, Nitro, Trifluormethyl, Trifluormethoxy oder eine Gruppe der Formel -NR³R⁴ stehen,
worin
R³ und R⁴ gleich oder verschieden sind und Wasserstoff, geradkettiges oder verzweigtes Alkyl mit bis zu 8 Kohlenstoffatomen oder Aryl mit 6 bis 10 Kohlenstoffatomen bedeuten,
- für geradkettiges oder verzweigtes Alkyl, Alkoxy oder Alkoxycarbonyl mit jeweils bis zu 12 Kohlenstoffatomen stehen, das gegebenenfalls durch Hydroxy, Halogen, Nitro, Cyano oder eine Gruppe der Formel -NR³R⁴ substituiert ist,
worin
R³ und R⁴ die oben angegebene Bedeutung haben,
- für Aryl mit 6 bis 10 Kohlenstoffatomen stehen, das gegebenenfalls durch Halogen, Hydroxy, Nitro, Cyano geradkettiges oder verzweigtes Alkyl, Alkoxy oder Alkoxycarbonyl mit jeweils bis zu 8 Kohlenstoffatomen oder durch eine Gruppe der Formel -NR³R⁴ substituiert ist,
worin
R³ und R⁴ die oben angegebene Bedeutung haben,
R¹
- für Cycloalkyl mit 3 bis 14 Kohlenstoffatomen steht, das gegebenenfalls durch geradkettiges oder verzweigtes Alkyl mit bis zu 8 Kohlenstoffatomen substituiert ist,
R²
- für Wasserstoff oder
- für geradkettiges oder verzweigtes Alkyl mit bis zu 10 Kohlenstoffatomen steht, das gegebenenfalls durch Hydroxy, Alkoxy mit bis zu 8 Kohlenstoffatomen, Halogen oder durch Cycloalkyl mit 3 bis 8 Kohlenstoffatomen oder Aryl mit 6 bis 10 Kohlenstoffatomen substituiert ist, das seinerseits durch geradkettiges oder verzweigtes Alkyl mit bis zu 8 Kohlenstoffatomen, Halogen, Nitro, Hydroxy oder Cyano substituiert sein kann, oder
- für Cycloalkyl mit 3 bis 8 Kohlenstoffatomen steht, das gegebenenfalls durch geradkettiges oder verzweigtes Alkyl mit bis zu 8 Kohlenstoffatomen substituiert ist, oder
- für ein Alkalimetall steht,
X
- für eine Gruppe der Formel -SO₂-R⁵ steht,
worin
R⁵
- Trifluormethyl oder geradkettiges oder verzweigtes Alkyl mit bis zu 10 Kohlenstoffatomen bedeutet, das gegebenenfalls durch Hydroxy, Halogen, Cyano, Alkoxy oder Alkoxycarbonyl mit jeweils bis zu 8 Kohlenstoffatomen oder durch Aryl mit 6 bis 10 Kohlenstoffatomen substituiert ist, welches seinerseits durch Halogen, Nitro, Cyano oder geradkettiges oder verzweigtes Alkyl oder Alkoxy mit jeweils bis zu 8 Kohlenstoffatomen substituiert sein kann, oder
- Aryl mit 6 bis 10 Kohlenstoffatomen bedeutet, das gegebenenfalls durch Halogen, Nitro, Cyano, Hydroxy, geradkettiges oder verzweigtes Alkyl, Alkoxy oder Alkoxycarbonyl mit jeweils bis zu 8 Kohlenstoffatomen, Trifluormethyl oder Trifluormethoxy substituiert ist,
und deren physiologisch unbedenkliche Salze dadurch gekennzeichnet, daß man zunächst die Carbonsäuren der allgemeinen Formel (II) über die Stufen der Säurehalogenide oder -anhydride nach üblichen Methoden in die entsprechenden Säureamide der allgemeinen Formel (IV) 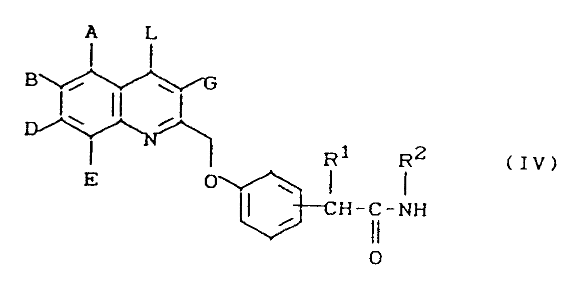 in welcher
A, B, D, E, L, G, R¹ und R² die oben angegebene Bedeutung haben,
überführt und in einem zweiten Schritt mit Sulfonsäurehalogeniden der allgemeinen Formel (V)
X-Hal (V)
in welcher
X
- in diesem Fall für die Gruppe -SO₂R⁵ steht, worin R⁵ die oben angegebene Bedeutung hat,
und
Hal für Fluor, Chlor, Brom oder Jod steht,
in inerten Lösemitteln sulfoniert.

3. Verwendung von (Chinolin-2-yl-methoxy)phenylacyl-sulfonamide und Cyanamide der allgemeinen Formel I nach Anspruch 1
worin
A, B, D, E, L, G, R¹, R² und X die in Anspruch 1 angegebene Bedeutung haben
zur Herstellung von Arzneimitteln.

## Claims (Claims for the following Contracting State(s): AT, BE, CH, DE, DK, FR, GB, GR, IT, LI, NL, LU, SE)

1. (Quinolin-2-yl-methoxy)phenylacyl-sulphonamides and -cyanamides of the general formula 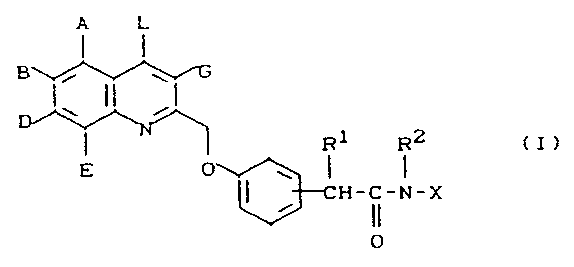 in which
A, B, D, E, L and G are identical or different and
- represent hydrogen, hydroxyl, halogen, carboxyl, nitro, trifluoromethyl, trifluoromethoxy or a group of the formula -NR³R⁴,
in which
R³ and R⁴ are identical or different and denote hydrogen, straight-chain or branched alkyl having up to 8 carbon atoms or aryl having 6 to 10 carbon atoms,
- represent straight-chain or branched alkyl, alkoxy or alkoxycarbonyl in each case having up to 12 carbon atoms, and each of which is optionally substituted by hydroxyl, halogen, nitro, cyano or a group of the formula -NR³R⁴,
in which
R³ and R⁴ have the abovementioned meaning,
- represent aryl having 6 to 10 carbon atoms, which is optionally substituted by halogen, hydroxyl, nitro, cyano, straight-chain or branched alkyl, alkoxy or alkoxycarbonyl in each case having up to 8 carbon atoms or by a group of the formula -NR³R⁴,
in which
R³ and R⁴ have the abovementioned meaning,
R¹
- represents cycloalkyl having 3 to 14 carbon atoms, which is optionally substituted by straight-chain or branched alkyl having up to 8 carbon atoms,
R²
- represents hydrogen or
- represents straight-chain or branched alkyl having up to 10 carbon atoms, which is optionally substituted by hydroxyl, alkoxy having up to 8 carbon atoms, halogen or by cycloalkyl having 3 to 8 carbon atoms or aryl having 6 to 10 carbon atoms, which in turn may be substituted by straight-chain or branched alkyl having up to 8 carbon atoms, halogen, nitro, hydroxyl or cyano, or
- represents cycloalkyl having 3 to 8 carbon atoms which is optionally substituted by straight-chain or branched alkyl having up to 8 carbon atoms, or
- represents an alkali metal,
X
- represents a group of the formula -SO₂-R⁵,
in which
R⁵
- denotes trifluoromethyl or straight-chain or branched alkyl having up to 10 carbon atoms, which is optionally substituted by hydroxyl, halogen, cyano, alkoxy or alkoxycarbonyl in each case having up to 8 carbon atoms or by aryl having 6 to 10 carbon atoms, which in turn may be substituted by halogen, nitro, cyano or straight-chain or branched alkyl or alkoxy in each case having up to 8 carbon atoms, or
- denotes aryl having 6 to 10 carbon atoms, which is optionally substituted by halogen, nitro, cyano, hydroxyl, straight-chain or branched alkyl, alkoxy or alkoxycarbonyl in each case having up to 8 carbon atoms, trifluoromethyl or trifluoromethoxy,
or
X
- represents cyano
and their physiologically acceptable salts.

2. (Quinolin-2-yl-methoxy)phenylacyl-sulphonamides and -cyanamides of the formula (I) according to Claim 1
in which
A, B, D, E, L and G are identical or different and
- represent hydrogen, hydroxyl, fluorine, chlorine, bromine, carboxyl, nitro, trifluoromethyl, trifluoromethoxy or a group of the formula -NR³R⁴,
in which
R³ and R⁴ are identical or different and denote hydrogen, straight-chain or branched alkyl having up to 6 carbon atoms or phenyl,
- represent straight-chain or branched alkyl, alkoxy or alkoxycarbonyl in each case having up to 10 carbon atoms, and each of which is optionally substituted by hydroxyl, fluorine, chlorine, bromine, nitro, cyano or a group of the formula -NR³R⁴,
in which
R³ and R⁴ have the abovementioned meaning,
- represent phenyl which is optionally substituted by fluorine, chlorine, bromine, hydroxyl, nitro, cyano, straight-chain or branched alkyl, alkoxy or alkoxycarbonyl in each case having up to 6 carbon atoms or by a group of the formula -NR³R⁴,
in which
R³ and R⁴ have the abovementioned meaning,
R¹
- represents cyclopropyl, cyclobutyl, cyclopentyl, cyclohexyl, cycloheptyl, cyclooctyl, cyclodecyl or cyclododecyl, each of which is optionally substituted by straight-chain or branched alkyl having up to 6 carbon atoms,
R²
- represents hydrogen or
- represents straight-chain or branched alkyl having up to 8 carbon atoms, which is optionally substituted by hydroxyl, alkoxy having up to 6 carbon atoms, fluorine, chlorine, bromine, cyclopropyl, cyclobutyl, cyclopentyl, cyclohexyl, cycloheptyl or phenyl, which in turn may be substituted by straight-chain or branched alkyl having up to 6 carbon atoms, fluorine, chlorine or bromine, or
- represents cyclopropyl, cyclohexyl, cyclopentyl, cyclohexyl or cycloheptyl, each of which is optionally substituted by straight-chain or branched alkyl having up to 6 carbon atoms, or
- represents sodium or potassium,
X
- represents a group of the formula -SO₂-R⁵,
in which
R⁵
- denotes trifluoromethyl or straight-chain or branched alkyl having up to 8 carbon atoms, which is optionally substituted by fluorine, chlorine, bromine, alkoxycarbonyl in each case having up to 6 carbon atoms or by phenyl, which in turn may be substituted by fluorine, chlorine, bromine or by straight-chain or branched alkyl or alkoxy in each case having up to 6 carbon atoms, or
- denotes phenyl which is optionally substituted by fluorine, chlorine, bromine, nitro, cyano, straight-chain or branched alkyl, alkoxy or alkoxycarbonyl in each case having up to 6 carbon atoms or trifluoromethyl,
or
X
- represents cyano
and their physiologically acceptable salts.

3. (Quinolin-2-yl-methoxy)phenylacyl-sulphonamides and -cyanamides of the formula (I) according to Claim 1
in which
A, B, D, E, L and G are identical or different and
- represent hydrogen, fluorine, chlorine, bromine, nitro or trifluoromethyl,
- represent methyl, ethyl, propyl, isopropyl, butyl or tert.butyl,
R¹
- represents cyclopropyl, cyclobutyl, cyclopentyl, cyclohexyl, cycloheptyl, cyclooctyl, cyclodecyl or cyclododecyl, each of which is optionally substituted by methyl, ethyl, propyl or isopropyl,
R²
- represents hydrogen or
- represents straight-chain or branched alkyl having up to 6 carbon atoms, which is optionally substituted by cyclopropyl, cyclopentyl, cyclohexyl or cycloheptyl,
- represents cyclopropyl, cyclopentyl, cyclohexyl or cycloheptyl, or
- represents sodium,
X
- represents a group of the formula -SO₂-R⁵,
in which
R⁵
- denotes trifluoromethyl, straight-chain or branched alkyl having up to 6 carbon atoms, which is optionally substituted by phenyl which in turn is substituted by fluorine, chlorine or by straight-chain or branched alkyl having up to 4 carbon atoms, or
- denotes phenyl which may optionally be substituted by fluorine, chlorine or straight-chain or branched alkyl having up to 4 carbon atoms,
or
X
- represents cyano
and their physiologically acceptable salts.

4. (Quinolin-2-yl-methoxy)phenylacyl-sulphonamides and -cyanamides according to Claims 1-3 for combating diseases.

5. Process for the preparation of (quinolin-2-yl-methoxy)phenylacyl-sulphonamides and -cyanamides of the general formula 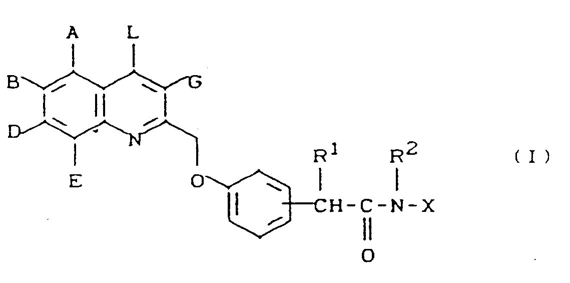 in which
A, B, D, E, L, G, R¹, R² and X have the meaning given in Claims 1 to 3,
and their physiologically acceptable salts,
characterized in that
carboxylic acids of the general formula (II) 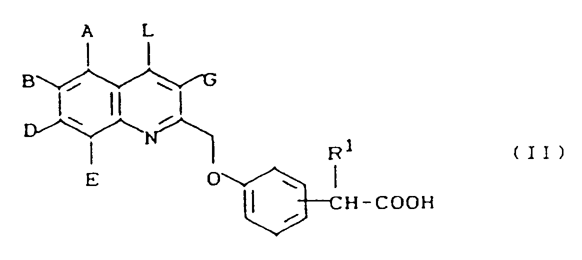 are amidated with amides of the general formula (III) 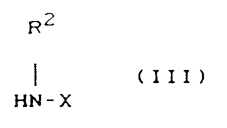 in inert solvents, if appropriate in the presence of dehydrating agents.

6. Process for the preparation of (quinolin-2-yl-methoxy)phenylacyl-sulphonamides of the general formula (Ia) 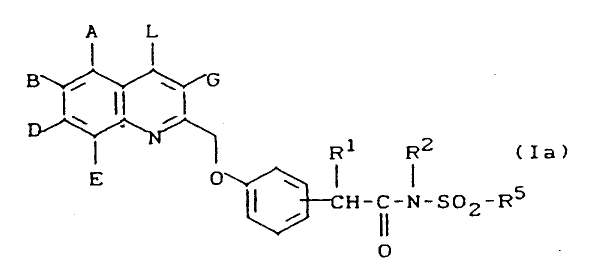 in which
A, B, D, E, L, G, R¹, R² and X have the meaning given in claims 1-3,
but X does not represent cyano
and their physiologically acceptable salts, characterized in that
the carboxylic acids of the general formula (II) are first converted via the acid halide or anhydride stages according to customary methods to the corresponding acid amides of the general formula (IV) 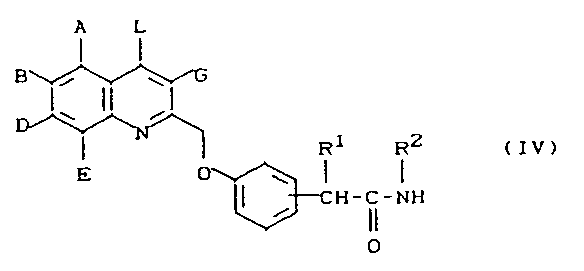 and in a second step are sulphonated with sulphonyl halides of the general formula (V)
X-Hal (V)
in which
X
- in this case represents the group -SO₂R⁵, in which R⁵ has the abovementioned meaning,
and
Hal represents fluorine, chlorine, bromine or iodine,
in inert solvents.

7. Medicaments containing at least one (quinolin-2-yl-methoxy)phenylacyl-sulphonamide and -cyanamide according to Claims 1-3.

8. Method for the production of a medicament according to Claim 6, characterized in that compounds according to Claim 1 are brought into a suitable form for administration, if appropriate with the aid of customary auxiliaries and excipients.

9. Use of (quinolin-2-yl-methoxy)phenylacyl-sulphonamides and -cyanamides according to Claim 1 for the production of medicaments.

10. (+)-Enantiomers and (-)-enantiomers of substituted (quinolin-2-yl-methoxy)phenylacyl-sulphonamides and -cyanamides according to Claims 1-3.

11. (+)-N-[1-[4-(Quinolin-2-yl-methoxy)-phenyl]-1-cycloheptyl]acetyl-methanesulphonamide.

12. (-)-N-[1-[4-(Quinolin-2-yl-methoxy)-phenyl]-1-cycloheptyl]acetyl-methane-sulphonamide.

## Claims (Claims for the following Contracting State(s): ES)

1. Process for the preparation of (quinolin-2-yl-methoxy)phenylacyl-sulphonamides and -cyanamides of the general formula 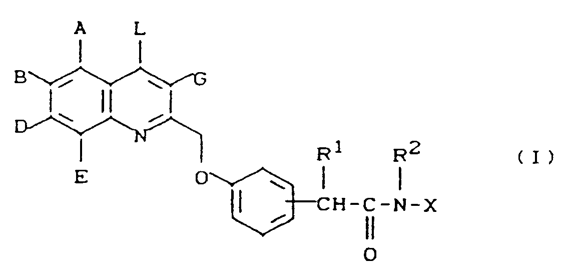 in which
A, B, D, E, L, and G are identical or different and
- represent hydrogen, hydroxyl, halogen, carboxyl, nitro, trifluoromethyl, trifluoromethoxy or a group of the formula -NR³R⁴,
in which
R³ and R⁴ are identical or different and denote hydrogen, straight-chain or branched alkyl having up to 8 carbon atoms or aryl having 6 to 10 carbon atoms,
- represent straight-chain or branched alkyl, alkoxy or alkoxycarbonyl in each case having up to 12 carbon atoms, and each of which is optionally substituted by hydroxyl, halogen, nitro, cyano or a group of the formula -NR³R⁴,
in which
R³ and R⁴ have the abovementioned meaning,
- represent aryl having 6 to 10 carbon atoms, which is optionally substituted by halogen, hydroxyl, nitro, cyano, straight-chain or branched alkyl, alkoxy or alkoxycarbonyl in each case having up to 8 carbon atoms or by a group of the formula -NR³R⁴,
in which
R³ and R⁴ have the abovementioned meaning,
R¹
- represents cycloalkyl having 3 to 14 carbon atoms, which is optionally substituted by straight-chain or branched alkyl having up to 8 carbon atoms,
R²
- represents hydrogen or
- represents straight-chain or branched alkyl having up to 10 carbon atoms, which is optionally substituted by hydroxyl, alkoxy having up to 8 carbon atoms, halogen or by cycloalkyl having 3 to 8 carbon atoms or aryl having 6 to 10 carbon atoms, which in turn may be substituted by straight-chain or branched alkyl having up to 8 carbon atoms, halogen, nitro, hydroxyl or cyano, or
- represents cycloalkyl having 3 to 8 carbon atoms which is optionally substituted by straight-chain or branched alkyl having up to 8 carbon atoms, or
- represents an alkali metal,
X
- represents a group of the formula -SO₂-R⁵,
in which
R⁵
- denotes trifluoromethyl or straight-chain or branched alkyl having up to 10 carbon atoms, which is optionally substituted by hydroxyl, halogen, cyano, alkoxy or alkoxycarbonyl in each case having up to 8 carbon atoms or by aryl having 6 to 10 carbon atoms, which in turn may be substituted by halogen, nitro, cyano or straight-chain or branched alkyl or alkoxy in each case having up to 8 carbon atoms, or
- denotes aryl having 6 to 10 carbon atoms, which is optionally substituted by halogen, nitro, cyano, hydroxyl, straight-chain or branched alkyl, alkoxy or alkoxycarbonyl in each case having up to 8 carbon atoms, trifluoromethyl or trifluoromethoxy,
or
X
- represents cyano
and their physiologically acceptable salts, characterized in that
carboxylic acids of the general formula (II) 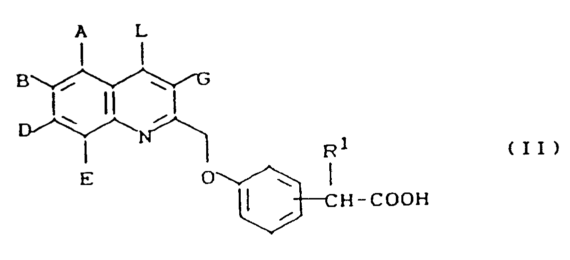 in which
A, B, D, E, L, G and R¹ have the abovementioned meaning,
are amidated with amides of the general formula (III) 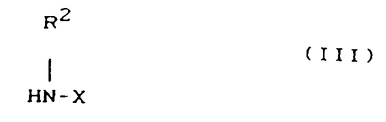 in which
R² and X have the abovementioned meaning,
in inert solvents, if appropriate in the presence of dehydrating agents.

2. Process for the preparation of (quinolin-2-yl-methoxy)phenylacyl-sulphonamides of the general formula (Ia) 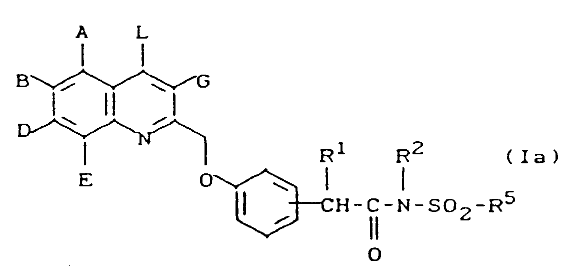 in which
A, B, D, E, L, and G are identical or different and
- represent hydrogen, hydroxyl, halogen, carboxyl, nitro, trifluoromethyl, trifluoromethoxy or a group of the formula -NR³R⁴,
in which
R³ and R⁴ are identical or different and denote hydrogen, straight-chain or branched alkyl having up to 8 carbon atoms or aryl having 6 to 10 carbon atoms,
- represent straight-chain or branched alkyl, alkoxy or alkoxycarbonyl in each case having up to 12 carbon atoms, and each of which is optionally substituted by hydroxyl, halogen, nitro, cyano or a group of the formula -NR³R⁴,
in which
R³ and R⁴ have the abovementioned meaning,
- represent aryl having 6 to 10 carbon atoms, which is optionally substituted by halogen, hydroxyl, nitro, cyano, straight-chain or branched alkyl, alkoxy or alkoxycarbonyl in each case having up to 8 carbon atoms or by a group of the formula -NR³R⁴,
in which
R³ and R⁴ have the abovementioned meaning,
R¹
- represents cycloalkyl having 3 to 14 carbon atoms, which is optionally substituted by straight-chain or branched alkyl having up to 8 carbon atoms,
R²
- represents hydrogen or
- represents straight-chain or branched alkyl having up to 10 carbon atoms, which is optionally substituted by hydroxyl, alkoxy having up to 8 carbon atoms, halogen or by cycloalkyl having 3 to 8 carbon atoms or aryl having 6 to 10 carbon atoms, which in turn may be substituted by straight-chain or branched alkyl having up to 8 carbon atoms, halogen, nitro, hydroxyl or cyano, or
- represents cycloalkyl having 3 to 8 carbon atoms which is optionally substituted by straight-chain or branched alkyl having up to 8 carbon atoms, or
- represents an alkali metal,
X
- represents a group of the formula -SO₂-R⁵,
in which
R⁵
- denotes trifluoromethyl or straight-chain or branched alkyl having up to 10 carbon atoms, which is optionally substituted by hydroxyl, halogen, cyano, alkoxy or alkoxycarbonyl in each case having up to 8 carbon atoms or by aryl having 6 to 10 carbon atoms, which in turn may be substituted by halogen, nitro, cyano or straight-chain or branched alkyl or alkoxy in each case having up to 8 carbon atoms, or
- denotes aryl having 6 to 10 carbon atoms, which is optionally substituted by halogen, nitro, cyano, hydroxyl, straight-chain or branched alkyl, alkoxy or alkoxycarbonyl in each case having up to 8 carbon atoms, trifluoromethyl or trifluoromethoxy,
and their physiologically acceptable salts, characterized in that
the carboxylic acids of the general formula (II) are first converted via the acid halide or anhydride stages according to customary methods to the corresponding acid amides of the general formula (IV) 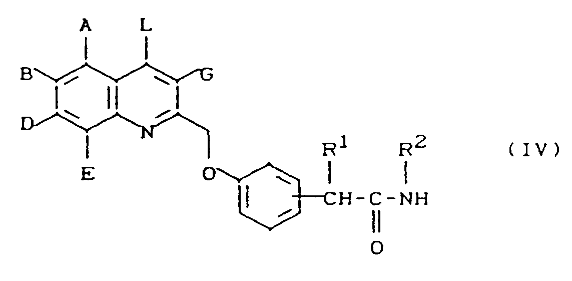 in which
A, B, D, E, L, G, R¹ and R² have the abovementioned meaning,
and in a second step are sulphonated with sulphonyl halides of the general formula (V)
X-Hal (V)
in which
X
- in this case represents the group -SO₂R⁵, in which R⁵ has the abovementioned meaning,
and
Hal represents fluorine, chlorine, bromine or iodine,
in inert solvents.

3. Use of (quinolin-2-yl-methoxy)phenylacyl-sulphonamides and -cyanamides of the general formula I according to Claim 1
in which
A, B, D, E, L, G, R¹, R² and X have the meaning given in Claim 1
for the production of medicaments.

## Revendications (Revendications pour l'(les) Etat(s) contractant(s) suivant(s): AT, BE, CH, DE, DK, FR, GB, GR, IT, LI, NL, LU, SE)

1. (Quinoléine-2-yl-méthoxy)phénylacylsulfonamides et -cyanamides, de formule générale : 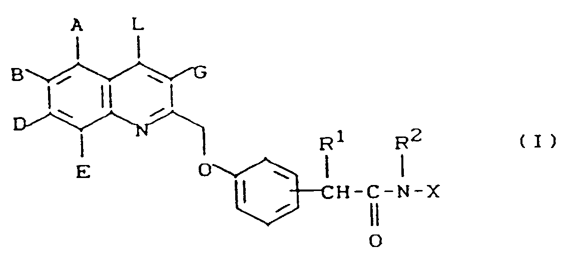 dans laquelle
A, B, D, E, L et G sont identiques ou différents et représentent chacun un atome d'hydrogène, un groupe hydroxy, halogéno, carboxy, nitro, trifluorométhyle, trifluorométhoxy ou un groupe de formule :
-NR³R⁴,
dans laquelle
R³ et R⁴ sont identiques ou différents et représentent chacun un atome d'hydrogène, un groupe alkyle linéaire ou ramifié ayant jusqu'à 8 atomes de carbone ou un groupe aryle ayant 6 à 10 atomes de carbone, un groupe alkyle, alcoxy ou alcoxycarbonyle, linéaire ou ramifié ayant chacun jusqu'à 12 atomes de carbone, qui est éventuellement substitué par un groupe hydroxy, halogéno, nitro, cyano ou par un groupe de formule :
-NR³R⁴,
dans laquelle
R³ et R⁴ ont le sens indiqué ci-dessus,
un groupe aryle ayant 6 à 10 atomes de carbone, qui est éventuellement substitué par de l'halogène, par un groupe hydroxy, nitro, cyano, par un groupe alkyle, alcoxy ou alcoxycarbonyle linéaire ou ramifié ayant chacun jusqu'à 8 atomes de carbone, ou par un groupe de formule :
-NR³R⁴,
dans laquelle
R³ et R⁴ ont le sens indiqué ci-dessus,
R¹ représente un groupe cycloalkyle ayant 3 à 14 atomes de carbone, qui est éventuellement substitué par un groupe alkyle linéaire ou ramifié ayant jusqu'à 8 atomes de carbone,
R² représente un atome d'hydrogène ou un groupe alkyle linéaire ou ramifié ayant jusqu'à 10 atomes de carbone, qui est éventuellement substitué par un groupe hydroxy, alcoxy ayant jusqu'à 8 atomes de carbone, halogéno ou par un groupe cycloalkyle ayant 3 à 8 atomes de carbone ou aryle ayant 6 à 10 atomes de carbone, lequel qui peut, pour sa part, être substitué par un groupe alkyle linéaire ou ramifié ayant jusqu'à 8 atomes de carbone, par de l'halogène, par un groupe nitro, hydroxy ou cyano, ou bien
R² représente un groupe cycloalkyle ayant 3 à 8 atomes de carbone, qui est éventuellement substitué par un groupe alkyle linéaire ou ramifié ayant jusqu'à 8 atomes de carbone, ou bien
R² représente un métal alcalin,
X représente un groupe de formule :
-SO₂-R⁵,
dans laquelle
R⁵ représente un groupe trifluorométhyle ou un groupe alkyle, linéaire ou ramifié, ayant jusqu'à 10 atomes de carbone, qui est éventuellement substitué par un groupe hydroxy, halogéno, cyano, alcoxy ou alcoxycarbonyle ayant chacun jusqu'à 8 atomes de carbone, ou par un groupe aryle ayant 6 à 10 atomes de carbone, lequel peut de son côté être substitué par de l'halogène, par un groupe nitro, cyano ou par un groupe alkyle ou alcoxy linéaire ou ramifié ayant chacun jusqu'à 8 atomes de carbone, ou bien
R⁵ représente un groupe aryle ayant 6 à 10 atomes de carbone, qui est éventuellement substitué par de l'halogène, par un groupe nitro, cyano, hydroxy, alkyle, alcoxy ou alcoxycarbonyle linéaire ou ramifié ayant chacun jusqu'à 8 atomes de carbone, par un groupe trifluorométhyle ou trifluorométhoxy, ou bien
X représente un groupe cyano,
et leurs sels physiologiquement inoffensifs.

2. (Quinoléine-2-yl-méthoxy)phénylacylsulfonamides et -cyanamides de formule (I) selon la revendication 1, dans laquelle :
A, B, D, E, L et G sont identiques ou différents et ils représentent chacun un atome d'hydrogène, un groupe hydroxy, un atome de fluor, de chlore, de brome, un groupe carboxy, nitro, trifluorométhyle, trifluorométhoxy ou un groupe de formule :
-NR³R⁴,
dans laquelle
R³ et R⁴ sont identiques ou différents et représentent chacun un atome d'hydrogène, un groupe alkyle linéaire ou ramifié ayant jusqu'à 6 atomes de carbone ou un groupe phényle,
- un groupe alkyle, alcoxy ou alcoxycarbonyle linéaire ou ramifié ayant chacun jusqu'à 10 atomes de carbone, qui est éventuellement substitué par un groupe hydroxy, par du fluor, du chlore, du brome, par un groupe nitro, cyano ou un groupe de formule :
-NR³R⁴,
dans laquelle
R³ et R⁴ ont le sens indiqué ci-dessus,
- un groupe phényle, qui est éventuellement substitué par du fluor, du chlore, du brome, par un groupe hydroxy, nitro, cyano, par un groupe alkyle, alcoxy ou alcoxycarbonyle linéaire ou ramifié ayant chacun jusqu'à 6 atomes de carbone ou par un groupe alkyle de formule :
-NR³R⁴,
dans laquelle
R³ et R⁴ ont le sens indiqué ci-dessus,
R¹ représente un groupe cyclopropyle, cyclobutyle, cyclopentyle, cyclohexyle, cycloheptyle, cyclooctyle, cyclodécyle ou cyclododécyle, qui est éventuellement substitué par un groupe alkyle linéaire ou ramifié ayant jusqu'à 6 atomes de carbone,
R² représente un atome d'hydrogène ou un groupe alkyle linéaire ou ramifié ayant jusqu'à 8 atomes de carbone, qui est éventuellement substitué par un groupe hydroxy, alcoxy ayant jusqu'à 6 atomes de carbone, par un atome de fluor, de chlore ou de brome, par un groupe cyclopropyle, cyclobutyle, cyclopentyle, cyclohexyle, cycloheptyle ou phényle, qui peut de son côté être substitué par un groupe alkyle linéaire ou ramifié ayant jusqu'à 6 atomes de carbone, par un atome de fluor, de chlore ou de brome, ou
R² représente un groupe cyclopropyle, cyclobutyle, cyclopentyle, cyclohexyle ou cycloheptyle, qui est éventuellement substitué par un groupe alkyle linéaire ou ramifié ayant jusqu'à 6 atomes de carbone, ou bien
R² représente un atome de sodium ou de potassium,
X représente un groupe de formule :
-SO₂-R⁵,
dans laquelle
R⁵ représente un groupe trifluorométhyle ou un groupe alkyle linéaire ou ramifié ayant jusqu'à 8 atomes de carbone, qui est éventuellement substitué par un atome de fluor, de chlore, de brome, par un groupe alcoxycarbonyle ayant chacun jusqu'à 6 atomes de carbone ou par un groupe phényle, lequel peut à son tour être substitué par un atome de fluor, de chlore ou de brome ou par un groupe alkyle ou alcoxy linéaire ou ramifié ayant chacun jusqu'à 6 atomes de carbone, ou bien
R⁵ représente un groupe phényle, qui est éventuellement substitué par un atome de fluor, de chlore ou de brome ou par un groupe nitro, cyano, alkyle, alcoxy ou alcoxycarbonyle linéaire ou ramifié ayant chacun jusqu'à 6 atomes de carbone ou par un groupe trifluorométhyle, ou bien,
X représente un groupe cyano,
et leurs sels physiologiquement inoffensifs.

3. (Quinoléne-2-yl-méthoxy)phénylacylsulfonamides et -cyanamides de formule (I) selon la revendication 1, dans laquelle :
A, B, D, E, L et G sont identiques ou différents et ils représentent :
un atome d'hydrogène, de fluor, de chlore, de brome, un groupe nitro ou trifluorométhyle, un groupe méthyle, éthyle, propyle, isopropyle, butyle ou tert-butyle ;
R¹ représente un groupe cyclopropyle, cyclobutyle, cyclopentyle, cyclohexyle, cycloheptyle, cyclooctyle, cyclodécyle ou cyclododécyle qui est éventuellement substitué par un groupe méthyle, éthyle, propyle ou isopropyle,
R² représente un atome d'hydrogène ou un groupe alkyle linéaire ou ramifié ayant jusqu'à 6 atomes de carbone, qui est éventuellement substitué par un groupe cyclopropyle, cyclopentyle, cyclohexyle ou cycloheptyle, un groupe cyclopropyle, cyclopentyle, cyclohexyle ou cycloheptyle, ou un atome de sodium,
X représente un groupe de formule :
-SO₂-R⁵,
dans laquelle
R⁵ représente un groupe trifluorométhyle, un groupe alkyle linéaire ou ramifié ayant jusqu'à 6 atomes de carbone, qui est éventuellement substitué par un groupe phényle, lequel est substitué pour sa part par un atome de fluor ou de chlore ou par un groupe alkyle linéaire ou ramifié ayant jusqu'à 4 atomes de carbone, ou
R⁵ représente un groupe phényle, qui peut éventuellement être substitué par du fluor, par du chlore ou par un groupe alkyle linéaire ou ramifié ayant jusqu'à 4 atomes de carbone, ou bien
X représente un groupe cyano,
et leurs sels physiologiquement inoffensifs.

4. (Quinoléine-2-yl-méthoxy)phénylacylsulfonamides et -cyanamides selon l'une des revendications 1 à 3, pour lutter contre des maladies.

5. Procédé pour préparer des (quinoléine-2-yl-méthoy)phénylacylsulfonamides et -cyanamides de formule générale : 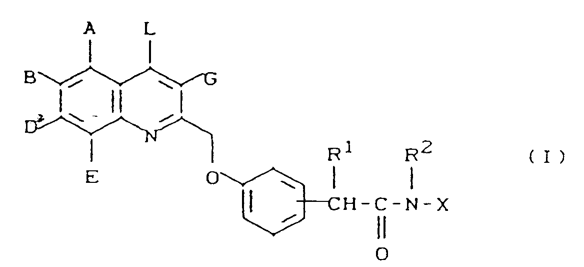 dans laquelle
A, B, D, L, G, R¹ R² et X ont le sens indiqué aux revendications 1 à 3, et leurs sels physiologiquement inoffensifs,
procédé caractérisé en ce qu'on amide des acides carboxyliques de formule générale (II) : 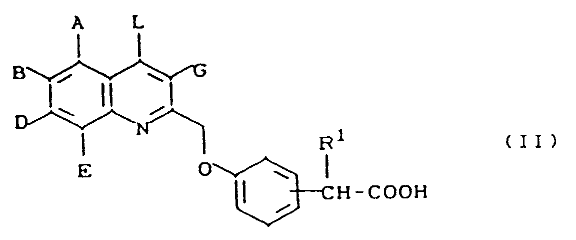 avec des amides de formule générale (III) : 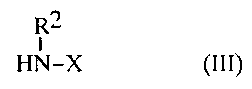 dans des solvants inertes, éventuellement en présence d'agents de déshydratation.

6. Procédé pour préparer des (quinoléine-2-yl-méthoxy)phénylacylsulfonamides de formule générale (Ia) : 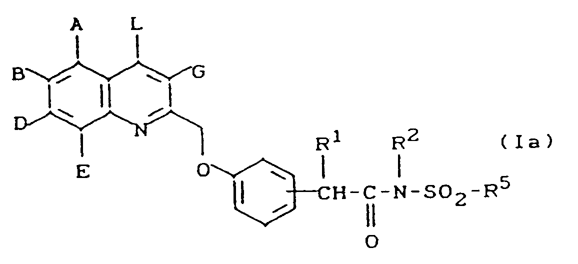 dans laquelle
A, B, D, E, L, G, R¹, R² et X ont le sens indiqué aux revendications 1 à 3,
X ne représentant cependant pas le groupe cyano,
et leurs sels physiologiquement inoffensifs, procédé caractérisé en ce qu'on transforme tout d'abord les acides carboxyliques de formule générale (II), en passant par les stades des halogénures d'acides ou des anhydrides d'acides, selon les méthodes connues, en les amides d'acides correspondants, de formule générale (IV) : 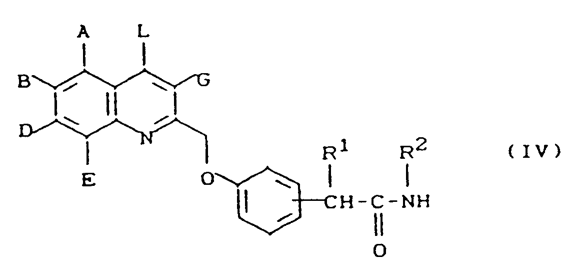 et, dans une seconde étape, on sulfone dans des solvants inertes avec des halogénures d'acides sulfoniques de formule générale (V) :
X-Hal (V)
dans laquelle
X dans ce cas représente le groupe :
-SO₂R⁵,
dans laquelle
R⁵ est le sens précité, et
Hal représente un atome de fluor, de chlore, de brome ou d'iode.

7. Médicament contenant au moins un (quinoléine-2-yl-méthoxy)phénylacylsulfonamide et -cyanamide selon l'une des revendications 1 à 3.

8. Procédé pour préparer un médicament selon la revendication 6, caractérisé en ce qu'on met sous une forme convenant bien pour une application des composés selon la formule (I), éventuellement à l'aide d'adjuvants et de supports, excipients ou véhicules usuels.

9. Utilisation des (quinoléine-2-yl-méthoy)phénylacylsulfonamides et -cyanamides selon la revendication 1 pour préparer des médicaments.

10. Enantiomères (+) et énantiomères (-) des (quinoléine-2-yl-méthoxy)phénylarylsulfonamide et -cyanamide substitués selon l'une des revendications 1 à 3.

11. Le (+)-N-{1-[4-(quinoléine-2-yl-méthoxy)phényl]-1-cycloheptyl}acétylméthanesulfonamide.

12. Le (-)-N-{1-[4-(quinoléine-2-yl-méthoxy)phényl]-1-cycloheptyl}acétylméthanesulfonamide.

## Revendications (Revendications pour l'(les) Etat(s) contractant(s) suivant(s): ES)

1. Procédé pour préparer des (quinoléine-2-yl-méthoxy)-phénylacylsulfonamides et -cyanamides de formule générale : 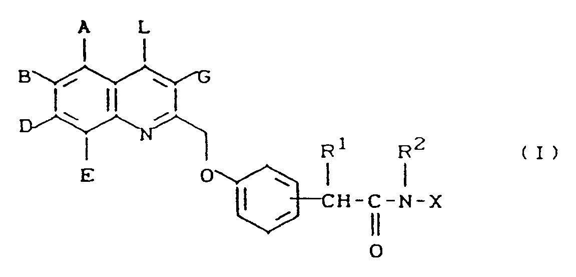 dans laquelle
A, B, D, E, L et G sont identiques ou différents et
ils représentent chacun un atome d'hydrogène, un groupe hydroxy, halogéno, carboxy, nitro, trifluorométhyle, trifluorométhoxy ou un groupe de formule :
-NR³R⁴,
dans laquelle
R³ et R⁴ sont identiques ou différents et représentent chacun un atome d'hydrogène, un groupe alkyle linéaire ou ramifié ayant jusqu'à 8 atomes de carbone ou aryle ayant 6 à 10 atomes de carbone,
- un groupe alkyle, alcoxy ou alcoxycarbonyle, linéaire ou ramifié ayant chacun jusqu'à 12 atomes de carbone, qui est éventuellement substitué par un groupe hydroxy, halogéno, nitro, cyano ou par un groupe de formule :
-NR³R⁴,
dans laquelle
R³ et R⁴ ont le sens indiqué ci-dessus,
- un groupe aryle ayant 6 à 10 atomes de carbone, qui est éventuellement substitué par de l'halogène, par un groupe hydroxy, nitro, cyano, par un groupe alkyle, alcoxy ou alcoxycarbonyle linéaire ou ramifié ayant chacun jusqu'à 8 atomes de carbone, ou par un groupe de formule :
-NR³R⁴,
dans laquelle
R³ et R⁴ ont le sens indiqué,
R¹ représente un groupe cycloalkyle ayant 3 à 14 atomes de carbone, qui est éventuellement substitué par un groupe alkyle linéaire ou ramifié ayant jusqu'à 8 atomes de carbone,
R² représente un atome d'hydrogène ou un groupe alkyle linéaire ou ramifié ayant jusqu'à 10 atomes de carbone, qui est éventuellement substitué par un groupe hydroxy, alcoxy ayant jusqu'à 8 atomes de carbone, par un halogène ou par un groupe cycloalkyle ayant 3 à 8 atomes de carbone ou par un groupe aryle ayant 6 à 10 atomes de carbone, qui peut être substitué pour sa part par un groupe alkyle linéaire ou ramifié ayant jusqu'à 8 atomes de carbone, par de l'halogène, par un groupe nitro, hydroxy ou cyano, ou bien
R² représente un groupe cycloalkyle ayant 3 à 8 atomes de carbone, qui est éventuellement substitué par un groupe alkyle linéaire ou ramifié ayant jusqu'à 8 atomes de carbone, ou bien
R² représente un métal alcalin,
X représente un groupe de formule :
-SO₂-R⁵,
dans laquelle
R⁵ représente un groupe trifluorométhyle ou un groupe alkyle, linéaire ou ramifié, ayant jusqu'à 10 atomes de carbone, qui est éventuellement substitué par un groupe hydroxy, halogéno, cyano, alcoxy ou alcoxycarbonyle ayant chacun jusqu'à 8 atomes de carbone, ou par un groupe aryle ayant 6 à 10 atomes de carbone, lequel peut de son côté être substitué par de l'halogène, par un groupe nitro, cyano ou par un groupe alkyle ou alcoxy linéaire ou ramifié ayant chacun jusqu'à 8 atomes de carbone, ou bien
R⁵ représente un groupe aryle ayant 6 à 10 atomes de carbone, qui est éventuellement substitué par de l'halogène, par un groupe nitro, cyano, hydroxy, par un groupe alkyle, alcoxy ou alcoxycarbonyle linéaire ou ramifié ayant chacun jusqu'à 8 atomes de carbone, par un groupe trifluorométhyle ou trifluorométhoxy, ou bien
X représente un groupe cyano,
et leurs sels physiologiquement inoffensifs,
procédé caractérisé en ce qu'on amide des acides carboxyliques de formule générale (II) : 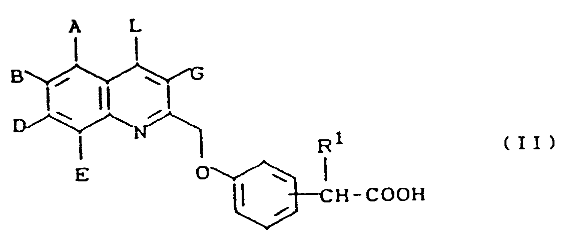 dans laquelle
A, B, D, E, L, G et R¹ ont le sens indiqué ci-dessus,
avec des amides de formule générale (III) : 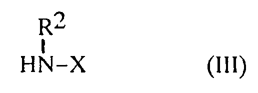 dans laquelle
R² et X ont le sens indiqué ci-dessus, en opérant dans des solvants inertes, éventuellement en présence d'agents de déshydratation.

2. Procédé de préparation de (quinoléine-2-yl-méthoxy)phénylacylsulfonamides de formule générale (Ia) : 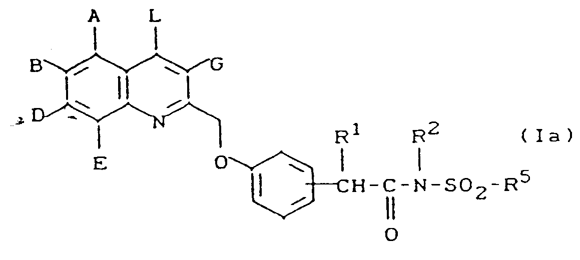 dans laquelle
A, B, D, E, L et G sont indiques ou différents et représentent chacun un atome d'hydrogène, un groupe hydroxy, halogéno, carboxy, nitro, trifluorométhyle, trifluorométhoxy ou un groupe de formule :
-NR³R⁴,
dans laquelle
R³ et R⁴ sont identiques ou différents et représentent chacun un atome d'hydrogène, un groupe alkyle linéaire ou ramifié ayant jusqu'à 8 atomes de carbone ou un groupe aryle ayant de 6 à 10 atomes de carbone,
- un groupe alkyle, alcoxy ou alcoxycarbonyle linéaire ou ramifié ayant chacun jusqu'à 12 atomes de carbone, qui est éventuellement substitué par un groupe hydroxy, halogéno, nitro, cyano ou par un groupe de formule :
-NR³R⁴,
dans laquelle
R³ et R⁴ ont le sens indiqué ci-dessus,
- un groupe aryle ayant 6 à 10 atomes de carbone, qui est éventuellement substitué par de l'halogène, par un groupe hydroxy, nitro, cyano, par un groupe alkyle, alcoxy ou alcoxycarbonyle linéaire ou ramifié ayant chacun jusqu'à 8 atomes de carbone ou par un groupe de formule :
-NR³R⁴,
dans laquelle
R³ et R⁴ ont le sens indiqué ci-dessus,
R¹ représente un groupe cycloalkyle ayant 3 à 14 atomes de carbone, qui est éventuellement substitué par un groupe alkyle linéaire ou ramifié ayant jusqu'à 8 atomes de carbone,
R² représente un atome d'hydrogène, ou un groupe alkyle linéaire ou ramifié ayant jusqu'à 10 atomes de carbone, qui est éventuellement substitué par un groupe hydroxy, alcoxy ayant jusqu'à 8 atomes de carbone, halogéno ou par un groupe cycloalkyle ayant 3 à 8 atomes de carbone ou par un groupe aryle ayant 6 à 10 atomes de carbone qui peut être substitué pour sa part par un groupe alkyle linéaire ou ramifié ayant jusqu'à 8 atomes de carbone, par de l'halogène, par un groupe nitro, hydroxy ou cyano, ou bien
R² représente un groupe cycloalkyle ayant 3 à 8 atomes de carbone, qui est éventuellement substitué par un groupe alkyle linéaire ou ramifié ayant jusqu'à 8 atomes de carbone, ou bien
R² représente un atome de métal alcalin, et
X représente un groupe de formule :
-SO₂-R⁵,
dans laquelle
R⁵ représente un groupe trifluorométhyle ou un groupe alkyle, linéaire ou ramifié, ayant jusqu'à 10 atomes de carbone, qui est éventuellement substitué par un groupe hydroxy, halogéno, cyano, alcoxy ou alcoxycarbonyle ayant chacun jusqu'à 8 atomes de carbone, ou par un groupe aryle ayant 6 à 10 atomes de carbone, lequel peut pour sa part être substitué par de l'halogène, par un groupe nitro, cyano ou par un groupe alkyle ou alcoxy linéaire ou ramifié ayant chacun jusqu'à 8 atomes de carbone, ou bien
R⁵ représente un groupe aryle ayant 6 à 10 atomes de carbone, qui est éventuellement substitué par de l'halogène, par un groupe nitro, cyano, hydroxy, par un groupe alkyle, alcoxy ou alcoxycarbonyle linéaire ou ramifié ayant chacun jusqu'à 8 atomes de carbone, par un groupe trifluorométhyle ou trifluorométhoxy, et leurs sels physiologiquement inoffensifs, procédé caractérisé en ce qu'on transforme tout d'abord les acides carboxyliques de formule générale (II), en passant par les stades des halogénures d'acides ou des anhydrides d'acides, selon les méthodes usuelles, en les amides d'acides correspondants, de formule générale (IV) : 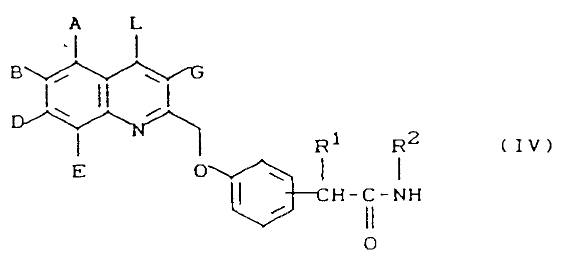 dans laquelle
A, B, D, E, L, G, R¹ et R² ont le sens indiqué ci-dessus,
et, dans une seconde étape, on sulfone dans des solvants inertes, avec des halogénures d'acides sulfoniques de formule générale (V):
X-Hal (V)
dans laquelle
X dans ce cas représente le groupe :
-SO₂R⁵,
dans laquelle
R⁵ a le sens indiqué ci-dessus, et
Hal représente un atome de fluor, de chlore, de brome ou d'iode.

3. Utilisation de (quinoléine-2-yl-méthoxy)phénylacylsulfonamides et -cyanamides de formule générale (I) selon la revendication 1, dans laquelle
A, B, D, E, L, G, R¹, R² et X ont le sens indiqué à la revendication 1, pour préparer des médicaments.
